# EUROPEAN PATENT SPECIFICATION

(11) **EP 0 515 536 B1**
(45) Date of publication and mention of the grant of the patent: **19.01.2000**
(21) Application number: 91905027.8
(22) Date of filing: 14.02.1991
(51) Int. Cl.: C12N 15/54, C12N 15/62, C12N 15/11, C12N 9/10, C12P 21/08, C12P 19/18, C12Q 1/68

(54) **METHODS AND PRODUCTS FOR THE SYNTHESIS OF OLIGOSACCHARIDE STRUCTURES ON GLYCOPROTEINS, GLYCOLIPIDS, OR AS FREE MOLECULES**
VERFAHREN UND PRODUKTE FÜR DIE SYNTHESE VON OLIGOSACCHARIDSTRUKTUREN AUF GLYKOPROTEINEN, GLYKOLIPIDEN ODER ALS FREIE MOLEKÜLE
PROCEDE ET PRODUITS DE SYNTHESE DE STRUCTURES D'OLIGOSACCHARIDE SUR DES GLYCOPROTEINES, DES GLYCOLIPIDES, OU EN TANT QUE MOLECULES LIBRES

(30) Priority: 14.02.1990 US 479858; 14.02.1990 US 480133; 12.12.1990 US 627621
(43) Date of publication of application: 02.12.1992
(73) Proprietor: THE REGENTS OF THE UNIVERSITY OF MICHIGAN, Ann Arbor, Michigan 48109-1248 (US)
(72) Inventor: LOWE, John B., Ann Arbor, MI 48105 (US)
(74) Representative: Le Guen, Gérard
(86) International application number: US9100899
(87) International publication number: WO9112340

(56) References cited:
- US-A- 4 634 665
- The Journal of Biological Chemistry, Volume 264, No. 6, issued 25 February 1989, L.K. ERNST et al., "Stable Expression of Blood Group H Determinants and GDP-L- fucose: beta-D-Galactoside 2-alpha-L-Fucosyltransferase in Mouse Cells after Transfection with Human DNA", pages 3436-3447; see pages 3437-3444.
- Proceeding of the National Academy of Sciences (USA), Vol. 86, issued November 1989, R.D. LARSEN et al., "Isolation of a cDNA Enocoding a Murine UDPgalactose: beta-D-galactosyl-1, 4-N-acetyl-D-glucosaminide alpha-1, 3-galactosyltransferase: Expression Cloning by Gene Transfer", pages 8227-8231; see entire document.

## Description

### Technical Field

The present invention relates to methods and products for the synthesis of oligosaccharide or polysaccharide structures, on glycoproteins, glycolipids, or as free molecules.

### Background Art

Carbohydrates are an important class of biological compounds. In cells carbohydrates function as structural components where they regulate viscosity, store energy, or are key comments of cell surfaces. Nearly all site specific intercellular interactions involve cell surface carbohydrates. For example, union of sperm and egg as well as the implantation of fertilized egg are both mediated by cell surface carbohydrates. Likewise, a number of proteins that function as cell adhesion molecules, including GMP-140, ELAM-1, and lymphocyte adhesion molecules like Mel-14, exhibit structural features that mimic lectins, and are thought to bind specific cell surface carbohydrate structures (Stoolman, Cell (1989) 56:907-910). Glycosylated proteins as tumor-associated antigens are now being used to identify the presence of numerous carcinomas. Even isolated oligosaccharides have been found to exhibit biological activity on their own.

Specific galactose glicosaccharides are known to inhibit the agglutination of uropathogenic coliform bacteria with red blood cells (U.S. Patent No. 4,521,592). Other oligosaccharides have been shown to possess potent antithrombic activity by increasing the levels of plasminogen activator (U.S. Patent No. 4,801,583). This same biological activity has been used, by binding oligosaccharides, in conjunction with an amino glycoprotein, to medical instruments to provide medical surfaces which have anticoagulation effects (U.S. Patent No. 4,810,784). Still other oligosaccharides have found utility as gram positive antibiotics and disinfectants (U.S. Patent Nos. 4,851,338 and 4,665,060). Further, oligosaccharides have been used as bacteria receptor sites in the diagnosis and identification of specific bacteria (U.S. Patent Nos. 4,657,849 and 4,762,824).

It is also well recognized that oligosaccharides have an influence on the protein or lipid to which the are conjugated (Rademacher et al, Ann. Rev. Biochem., (1988), 57: 785). Specific oligosaccharides have been shown to influence proteins, stability, rate of proteolysis, rate of in vivo clearance from the bloodstream, thermal stability and solubility. Changes in the oligosaccharide portion of cell surface carbohydrates have been noted in cells which have become cancerous. Other oligosaccharide changes have been detected during cell differentiation (Toone et al, Tetrahedron Report (1989) 45(17):5365-5422). As such, the significance of oligosaccharides to biological function cannot be understated.

The fundamental role of these materials in molecular biology has made them the object of considerable research, in particular, considerable efforts have been made in organic synthesis to synthesize these materials. Although synthetic approaches to making carbohydrates are quite developed, this technique suffers notable difficulties which relate to the selective protection and deprotection steps required in the available synthetic pathways. These difficulties, combined with difficulties associated with isolating and purifying carbohydrates, and determining their structures, has made it essentially impossible for synthetic organic chemistry to economically produce valuable carbohydrates.

Enzyme-mediated catalytic synthesis would offer dramatic advantages over the classical synthetic organic pathways, producing very high yields of carbohydrates (e.g., oligosaccharides and/or polysaccharides) economically, under mild conditions in aqueous solution, and without generating notable amounts of undesired side products. Such enzymes, which include glycosyltransferase, are however difficult to isolate, especially from urokaryotic, e.g., mammalian sources, because these proteins are only found in low concentrations, and are membrane-bound.

To date, standard molecular cloning approaches which require amino acid sequence information or anti-glycosyltransferase antibodies, have been successfully used to isolate just two eukaryotic, e.g., mammalian glycosyltransferase cDNAs, corresponding to β(1,4) galactosyltransferase (in 1986) and α(2,6)sialyltransferase (in 1987). In light of the above-noted considerable value of carbohydrates, there is accordingly a strongly felt need for an improved method for isolation of additional glycosyltransferase genes and cDNA, and for their use in carbohydrate synthesis.

### Disclosure of the Invention

Accordingly, it is an object of this invention to provide unmodified and modified isolated genes and cDNAs of glycosyltransferases. To use them, for example, in modifying cell surface oligosaccharide structure via gene transfer approaches or via in vitro glycosylation reactions.

ERNST et al. (1988), The journal of Biological Chemistry vol. 264 pp 3436-3447 disclose the expression in a mammalian cell line of DNA of human origin encoding an α-1,2-fucosyltransferase.

### Brief Description of the Drawings

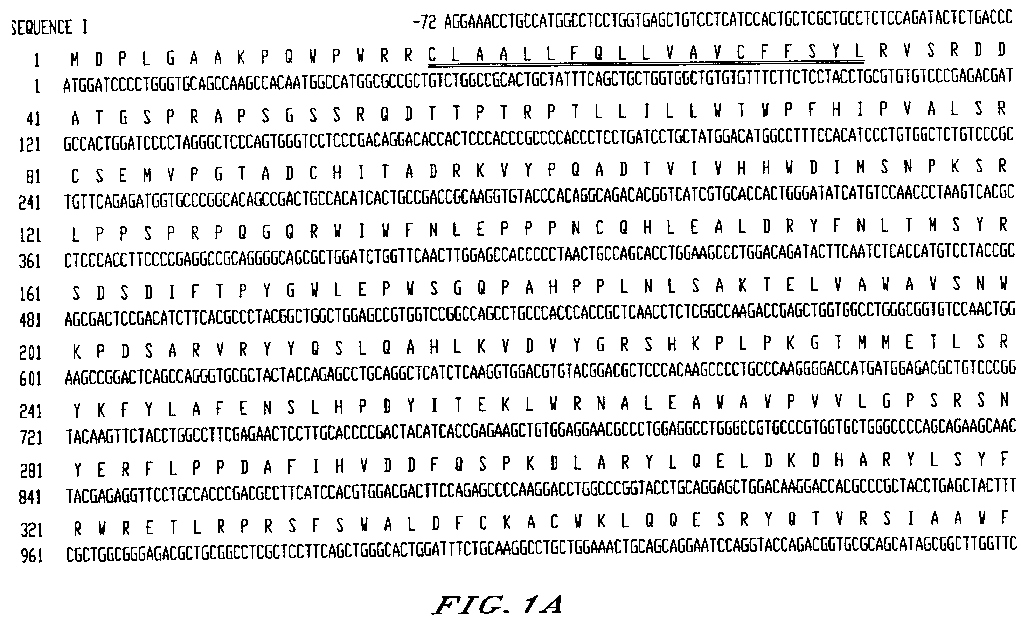

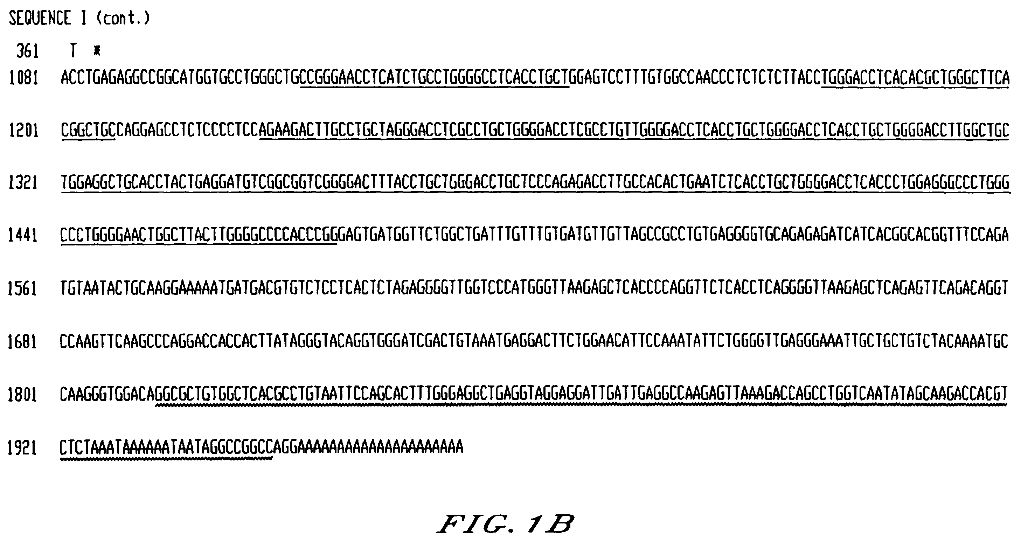

Figures 1, provides a DNA sequence (Sequences (I)), provided by the invention, encoding glycosyltransferases.
Figures 2 provide DNA sequences encoding a GDP-Fuc: [β-D-Gal (1, 4) ]-D-GlcNacα(1, 3)-fucosyltransferase.

### Abbreviations:

Sia; sialic acid
Gal; D-galactose
GalNac; D-N-acetylgalactosamine
Glc; D-glucose
GlcNAc; D-N-acetylglucosamine
Fuc; L-fucose
Man; D-mannose
IdUA; L-iduronic acid
GlcUA; D-glucuronic acid
Xyl; D-xylose
Ser; serine
Thr; threonine
Asn; asparagine

In one embodiment, the invention provides the DNA sequences set forth below.

The glycosyltransferases of the present invention correspond to: (1) at least amino acid positions 63 to 361 of sequence (I) and up to the whole sequence (I); or (4) at least from nucleotide position 2089 to nucleotide position 3156 (i.e., amino acid position 50 to 405) and up to the whole sequence (IV).

Sequence (I) encodes a protein sequence capable of functioning as a GDP-Fuc:[β-D-Gal(1,4/1,3)]-D-GlcNAc(/Glc) α (1,3/1,4)-fucosyltransferase. This protein is an enzyme that can be used to construct the oligosaccharide "ligand" for Endothelial Leukocyte Adhesion Molecule-1 (ELAM-1) that is disclosed in Applicant's co-pending U.S. Patent Application Serial No. 07/603,018, filed October 25, 1990, which is hereby incorporated by reference. This ligand is most probably the sialyl-Lewis x molecule. Also, this enzyme, when expressed by the cloned DNA sequence described here, functions within mammalian cells to generate de novo expression of specific cell surface glycoconjugate structures on those cells. Structures are recognized by antibodies against the following cell surface glycoconjugate structures:

| | |
|---|---|
| SSEA-1 or Lewis x | Galβ(1,4)[Fucα(1,3)]GlcNAc |
| sialyl-Lewis x | NeuAcα(2,3)Galβ(1,4)[Fucα(1,3)]GlcNA |
| Lewis a | Galβ(1,3)[Fucα(1,4)]GlcNAc |
| sialyl-Lewis a | NewAcα(2,3)Galβ(1,3)[Fucα(1,4)]GlcNAc. |

In the above DNA sequence (I), the sequence corresponding from amino acid position 43 to amino acid position 361 is functional, but a larger sequence of up to the whole sequence shown can be used.

This enzyme, when expressed by the cloned DNA sequence described here, functions in the enzymatic manner indicated in its name, when assayed in extracts prepared from cells that express the DNA sequence. The oligosaccharide product of this enzyme represents this fucose linked in alpha 1,3 configuration to neutral of α(2,3) sialylated "type II" acceptors, or fucose linked in alpha 1,4 configuration to neutral or α(2,3) sialylated "type I" acceptor of the following forms:

| | |
|---|---|
| SSEA-1 or Lewis x | Galβ(1,4)[Fucα(1,3)]GlcNAc |
| sialyl-Lewis x | NewAcα(2,3)Galβ(1,4)[Fucα(1,3)]GlcNAc |
| Lewis y | Fucα(1,2)Galβ(1,4)[Fucα(1.3)]GlcNAC |
| Lewis a | Galβ(1,3)[Fucα(1,4)]GlcNAc |
| sialyl-Lewis a | NeuAcα(2,3)Galβ(1,3)[Fucα(1,4)]GlcNAc |
| Lewis b | Fucα(1,2)Galβ(1,3)[Fucα(1,4)]GlcNAc. |

Throughout the remainder of this text, these products will be referred to as sub-terminal α(1,3) and α(1,4) fucose residues.

The catalytic domain of this enzyme has also been localized by expression studies. Each of the properties described above is outlined below. The enzymatic properties of the enzyme encoded by this cDNA, and chromosomal localization studies, indicate that this cDNA is the product of the human Lewis blood group locus.

This DNA sequence and the corresponding protein have the following uses:
(i.) Construction of animal cell lines with specific capabilities with respect to posttranslational modification of the oligosaccharides on cell-surface, intracellular, or secreted proteins or lipids by sub-terminal α(1,3) and α(1,4) fucose residues that represent the products of this enzyme (for the production of diagnostics and therapeutics).
   Specifically, the present cloned DNA sequence can be introduced by standard technologies into a mammalian cell line that does not normally express the cognate enzyme or its product (sub-terminal α(1,3) and α(1,4) fucose residues on oligosaccharides), and transcribed in that cell in the "sense" direction, to yield a cell line capable of expressing sub-terminal α(1,3) and α(1,4) fucose residues on oligosaccharides on cell-surface, intracellular, or secreted proteins or lipids.
   Alternatively, this cloned DNA sequence may be introduced by standard technologies into a mammalian cell line that does express the cognate enzyme and its product (sub-terminal α(1,3) and α(1,4) fucose residues), and transcribed in that cell in the "antisense" direction, to yield a cell line incapable of expressing sub-terminal α(1,3) and α(1,4) fucose residues on cell-surface, intracellular, or secreted proteins or lipids.
   Alternatively, the endogenous GDP-Fuc:[β-D--Gal(1,4/1,3)]-D-GlcNAc(/Glc)-α(1,3/1,4)--fucosyltransferase gene(s), in a mammalian cell expressing the cognate enzyme(s), can be inactivated with the DNA sequence described here by homologous recombination techniques, or by "anti-sense" oligonucleotide approaches based upon the DNA sequence described herein, or by dominant negative mutant fucosyltransferase sequences that inactivate endogenous GDP-Fuc:[β-D-Gal(1,4/1,3)]-D-GlcNAc(/Glc)α(1,3/1,4)-fucosyltransferase(s) and that may be derived via mutagenesis and genetic selection schemes, in conjunction with the sequence information in this text.
   This method can be used to construct animal cell lines that are suitable host cells for the production of diagnostic or therapeutic materials whose usefulness or efficacy depends upon the specific posttranslational modification determined by this cloned DNA sequence and its cognate enzyme. For example, it is known that the biological effectiveness of many therapeutic proteins or peptides, recombinant or otherwise, may depend critically upon the oligosaccharide structure(s) that are covalently attached to them. The structure of these oligosaccharides is primarily a function of the number and kind of glycosyltransferase enzymes that are found in the cell used to produce these therapeutic products.
   Animal cells and yeasts are competent to perform these glycosylation reactions; however, not all glycosyltransferase enzymes are produced by every animal cell or yeast, and therefore, some oligosaccharide structures (including sub-terminal α(1,3) and α(1,4) fucose residues generated by the enzyme encoded by the DNA sequence described here) are not produced by them.
   The converse is also true, namely, that producing cells may express a glycosyltransferase analagous to, or identical to, the GDP-Fuc: [β-D-Gal(1,4/1,3)]-D-GlcNAc(/Glc) α(1,3/1,4)-fucosyltransferase encoded by the DNA sequence described here. It is likely that sub-terminal α(1,3) and α(1,4) fucose residues alter the bioactivity (for better or for worse) of natural or recombinant therapeutic or diagnostic agents (glycoproteins or glycolipids) produced by mammalian or other eukaryotic hosts. Eukaryotic host cells that are used to produce these recombinant agents can be altered with the DNA sequence information and related information described in this invention, to add subterminal α(1,3) and α(1,4) fucose residues to the oligosaccharides on recombinant product by expressing all or part of the cloned sequences described here in the desired host. Alternatively, sub-terminal α(1, 3) and α(1,4) fucose residues may be eliminated from the product produced in these host cells by the use of transfected "anti-sense" vector constructs, recombination-based gene inactivation, "anti-sense" oligonucleotide approaches, or dominant negative mutant fucosyltransferases, outlined above.
   The old "methods" used for this process include an empirical approach to identify a cell line that does or does not express this particular enzyme or an enzyme that functions in a similar or identical manner, for the production of the appropriately modified recombinant or natural product. This is not always optimal since cell lines with this particular post-translation modification capabilities may not exist naturally, or may not be especially suited to high level production of an appropriately modified product. Alternatively, unwanted sub-terminal α(1,3) and α(1,4) fucose residues present on a therapeutic material produced by an empirically identified animal cell line must be removed chemically or enzymatically, a process that may be costly or inefficient.
   The advantages of using the cloned, functional DNA sequence described here in conjunction with the technologies outlined above, relative to these order methods, include the ability to construct lines that specifically lack the capability to generate sub-terminal α(1,3) and α(1,4) fucose residues on the oligosaccharides of glycoproteins and glycolipids; properly constructed, these cell lines will eliminate any need for chemical or enzymatic treatment of a therapeutic or diagnostic material to remove unwanted sub-terminal α(1,3) and α(1,4) fucose residues. Moreover, in the event that sub-terminal α(1,3) and α(1,4) fucose residues are found to be desirable for a particular diagnostic or therapeutic product produced by animal cells, cell lines may be engineered with the cloned DNA sequence described here to generate these residues.
(ii.) isolation of reagents suitable for efficient enzymatic synthesis and production of oligosaccharides (in enzyme reactors, for example).
   Oligosaccharides have therapeutic utility as immunomodulatory reagents in the field or organ transplantation. In particular, soluble and solid-phase oligosaccharides find use as therapeutic agents with which to block or ameliorate antibody-mediated organ transplant rejection in cases involving incompatibility due to differences in the major blood group antigen systems of the organ donor and the recipient, including the Lewis blood group system. Likewise, soluble oligosaccharides may find use as therapeutic agents that function by blocking attachment of bacterial, viral, or parasitic pathogens to glycoconjugate "receptors" found on the surface of the animal tissues that these pathogens invade.
   For example there is evidence that portions of the Lewis blood group oligosaccharide antigen (containing sub-terminal α(1,3) and α(1,4) fucose residues) serve as "receptors" for some forms of uropathogenic bacteria. Moreover, glycoconjugates, including sub-terminal α(1,3) and α(1,4) fucose residues, have been implicated in modulating adhesive events between cells, like mentrophil-ELAM-1 interaction, and between cells and their environment during developmental and differentiation processes. These events included binding of spermatozoa to eggs, and the initial events that mediate attachment of fertilized ova to the uterine wall at the beginning of implantation. These observations suggest, for example, the possibility that contraceptives for (biologically "natural") oligosaccharide molecules might exist. Oligosaccharide molecules contracted by this enzyme can disrupt mentrophil-ELAM interactions and thus function as anti-inflammatory agents.
   Currently, oligosaccharides containing subterminal α(1,3) and α(1,4) fucose residues are produced by chemical synthesis (a procedure that is inefficient and costly or both) or by isolation from natural sources (using costly and inefficient procedures that often require the processing of large quantities of animal or plant material, and the purification of the desired oligosaccharide from other contaminating oligosaccharides).
   The invention described here provides a mechanism to synthesize abundant quantities of purified GDP-Fuc:β-D-Gal (1,4) ]-D-GlcNAc(/Glc)α(1,3/1,4)-fucosyltransferase. This can be used to construct an enzyme bioreactor (enzyme in solution or immobilized on a solid phase matrix, for example via the protein-A moiety fused to the catalytic domain of the enzyme, as described in the accompanying manuscript) capable of enzymatic synthesis of structures containing subterminal α(1,3) and α(1,4) fucose residues.
   This is more efficient than approaches involving chemical synthesis of structures containing subterminal α(1,3) and α(1,4) fucose residues or their purification from natural sources, for a variety of reasons. One, the only chemicals necessary would be the enzyme substrates; these are easily obtained or synthesized. Two, enzymatic synthesis of such structures will produce only the desired product and the nucleotide diphosphate product of substrate hydrolysis. This latter chemical is found as the natural by-products of these reactions in animal cells, is relatively non-toxic, and may be easily separated from the oligosaccharide synthetic product. By contrast, chemical synthetic procedures typically generate numerous products of side reactions which must be removed, and which may be toxic as well, Similarly, purification of oligosaccharides from natural sources requires the removal of other contaminating oligosaccharides present in the natural material.
   Three, enzymatic catalysis is extraordinarily efficient; nearly complete conversion of substrate to product might be achieved. By contrast, chemical synthesis of sub-terminal α(1,3) and α(1,4) fucose residues on amides is a multi-step process; yields at each step may be much less than 100%, and the cumulative efficiency of current chemical synthesis procedures does not approach the efficiency possible with enzymatic synthesis. Similarly, purification of oligosaccharides with sub-terminal α(1,3) and α(1,4) fucose residues from natural materials can entail significant losses inherent to the purification procedures required to separate the desire oligosaccharide from contaminating, irrelevant oligosaccharides, with inefficient isolation of the desired oligosaccharide.
   Although the GDP-Fuc:[β-D-Gal(1,4/1,3)-D-GlcNAc(/Glc) α(1,3/1,4)-fucosyltransferase encoded by the DNA sequence described here may be from animal tissues for synthetic use, these purifications are themselves inefficient, primarily because the enzyme is typically present in very low abundance.
   This invention provides two mechanisms that provide for the abundant production of this enzyme. First, this may be done through the construction and viced selection of animal cells that produce relatively large quantities of the enzymes. Alternatively, this cloned nucleic acid sequence may then be used with standard recombinant DNA technologies to produce large quantities of glycosyltransferases in yeasts or in prokaryotic hosts. Furthermore, the sequence encoding this enzyme may be modified via standard molecular cloning schemes or mutagenesis to yield a recombinant fucosyltransferase with novel properties that make it more desirable than the wild-type enzyme.
   For example, modifications can be made to the enzyme that make it more stable, or more suitable for immobilization in a bioreactor.
(iii.) Isolation of reagents suitable for producing recombinant GDP-Fuc:[β-D-Gal(1,4/1,3)]-D-GlcNac(/Glc) α(1,3/1,4)-fucosyltransferase to be used directly as a research reagent, or to be used to generate antibodies against the GDP-Fuc:[β-D-Gal(1,4/1,3)]-D-GlcNAc(/Glc) α(1,3/1,4)-fucosyltransferase, for research applications.
   This invention provides two mechanisms for producing large quantities of this enzyme (see ii. above - i.e., specially constructed animal cells, or via natural or synthetic genes encoding these enzymes) which nay be used as a research tool with which to study the structures and functions of oligosaccharides and glycoproteins. Likewise, the enzyme produced by this method, or the nucleic acid sequence and derived protein sequence provided by this method, may be used to generate antibodies to this enzyme (via immunization with synthetic peptides whose sequences are derived from the cloned gene(s) or cDNA(s), or by immunization with the recombinant enzyme itself). These antibodies night also be used as research reagents to study the biosynthesis and processing of these enzymes, and can be used as an aid in their purification for all the uses described in this text.
(iv.) Antibodies to glycosyltransferases as diagnostic reagents.
   Aberrant expression of GDP-Fuc: [B-D-Gal (1,4/1,3)-D-GlcNac(/Glc) α(1,3/1,4) -fucosyltransferase has been associated with malignancy in humans, suggesting that this enzyme might serve as a tumor marker for early detection of malignancy involving a number of human tissues. Enzyme tumor markers have typically been assayed in body fluids by activity assays, which may be subject to non-specificity due to competing glycosyltransferase activity. These assays may also be insensitive since it is possible that inactive enzymes might be useful as tumor markers but would not be detected by enzyme activity assays.
   This invention provides a mechanism for generating antibodies to this enzyme (monoclonal and polyclonal antibodies against synthetic peptides constructed from information derived from cloned DNA sequence encoding GDP-Fuc:[β-D-Gal(1,4/1,3)]-D-GlcNaC(/Glc) α(1,3/1,4)-fucosyltransferase, or against the recombinant enzyme produced by eukaryotic or prokaryotic hosts). Antibodies specific for this GDP-Fuc: [β-D-Gal (1,4/1,3)]-D-GlcNac(/Glc) α(1,3/1,4)-fucosyltransferase produced could be used to detect and quantitate this glycosyltransferases in body fluids, with specificity and sensitivity exceeding enzyme activity assays, and with the possibility of serving as a tumor marker for early detection of malignancy.
(v.) Recombinant enzyme for use in screening natural and synthetic compounds for fucosyltransferase inhibitors or inactivators.
   A number of studies have noted an association between increased numbers of cell surface sub-terminal α(1,3) and α(1,4) fucose residues on oligosaccharides of a cell and the ability of that cell to metastasize in a malignant fashion. If there is a causal relationship, then drugs that inhibit the enzyme encoded by the sequence in this text could be active as anti-tumor agents. The reagents described in this text are useful for screening compounds for antifucosyltransferase activity, since the cloned sequence may be used with standard technique to produce relatively large amounts of pure fucosyltransferase. This aids in screening since the effects of potential inhibitors will be tested on a pure enzyme, without the confounding effects that may occur in whole cell extracts or with partially purified enzyme.
(vi.) Engineering of glycosyltransferase substrate specificity to generate novel glycoconjugate structures on secreted or cell-associated glycoconjugates.
   This invention provides a reagent (a cloned GDPFuc: [β-D-Gal (1,4/1,3)]-D-GlcNac(/Glc) α(1,3/1,4)-Fucosyltransferase cDNA) and the genetic selection method used to isolate it, that, when used with appropriate mutagenesis schemes, may allow the generation of mutant GDP-Fuc: [β-D-Gal (1,4/1,3) ]-D-GlcNac(/Glc)-α (1,3/1,4) -fucosyltransferases that generate glycosidic linkages different from that generated by the wild-type enzyme. These novel linkages may or may not be naturally occurring, and find utility as moieties that enhance bioactivity of the molecules to which they are attached.
   Alternatively, mutagenesis and selection approaches may be used to generate mutant GDP-Fuc:(β-D-Gal(1,4/1,3]-D-GlcNac(/Glc) α (1,3/1,4)-fucosyltransferases that act in a dominant negative fashion. The dominant negative mutants so generated can be used to inactivate endogenous glycosyltransferase activities when the product(s) of such an enzyme are not desired. Mutant GDP-Fuc: [β-D-Gal (1,4/1,3)]-D-GlcNac(/Glc) α (1,3/1,4)-fucosyltransferases can also be generated, for example, that function as fucosidases that hydrolyze various sugar linkages (fucose, mannose, or others) from oligosaccharides in vitro and in vivo.
(vii.) Genotyping individuals for the Lewis locus.
   Restriction fragment length polymorphisms within or linked to the gene corresponding to this cloned cDNA may be used to genotype individuals for the Lewis locus. This can find utility with respect to organ transplantation procedures, or as a measure of susceptibility to infections caused by pathogens that may use blood group structures as receptors for invasion (as in urinary tract infections, for example).

Other features of this invention will become apparent in the course of the following description of exemplary embodiments which are given for illustration of the invention and are not intended to be limiting thereof.

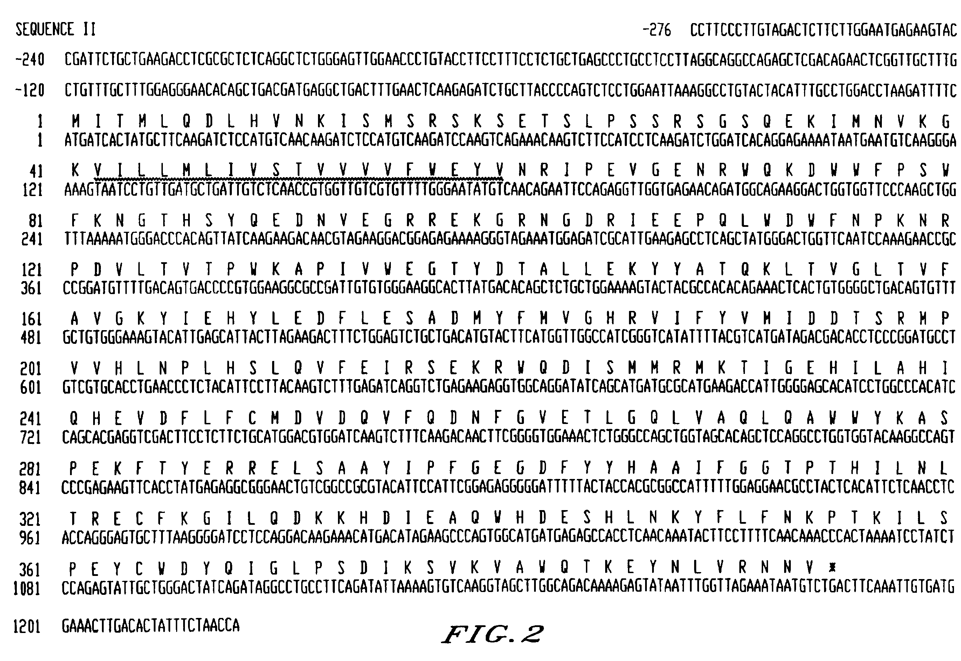

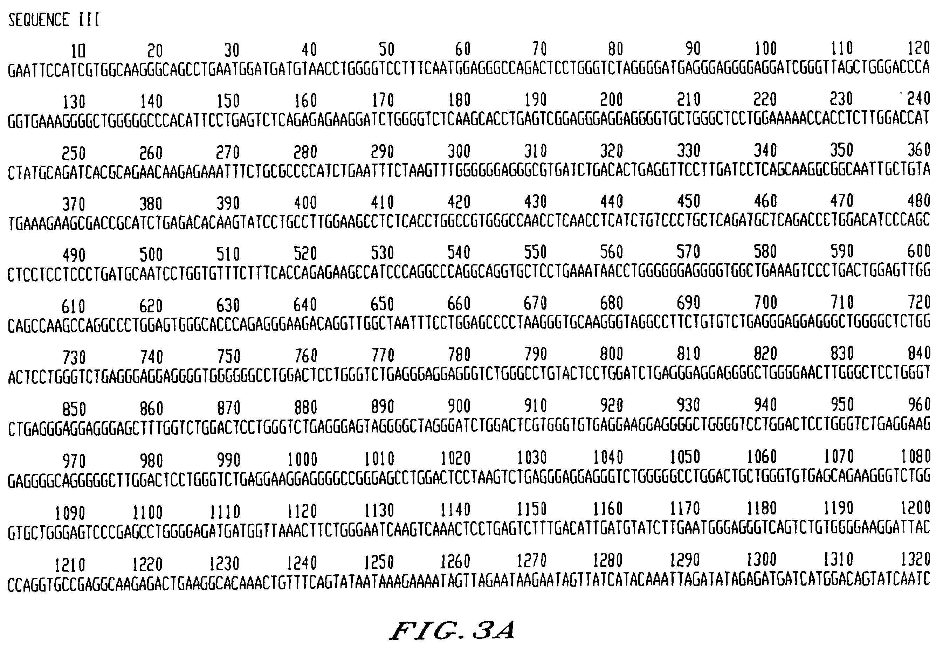

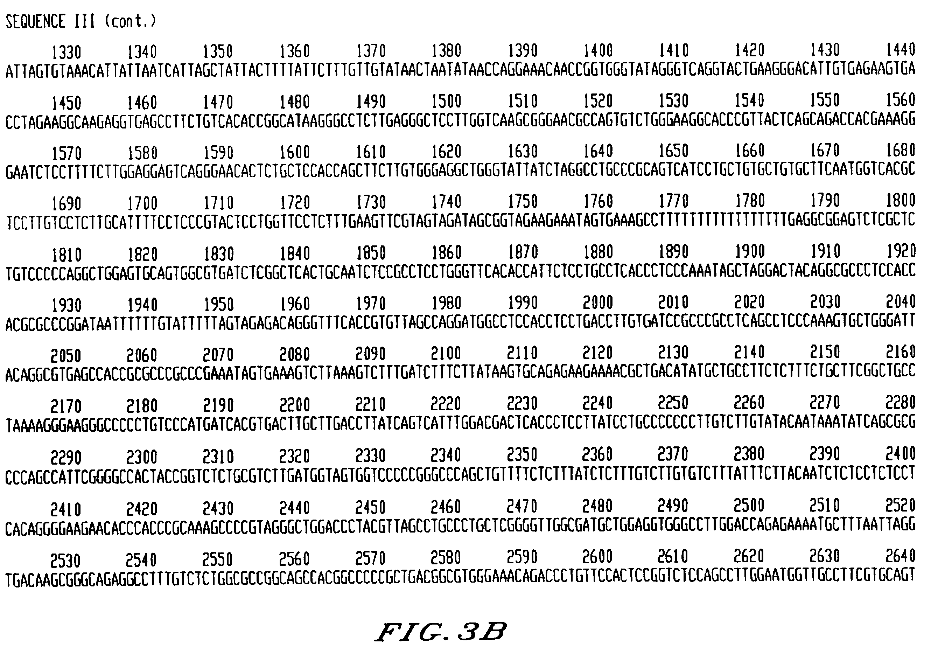

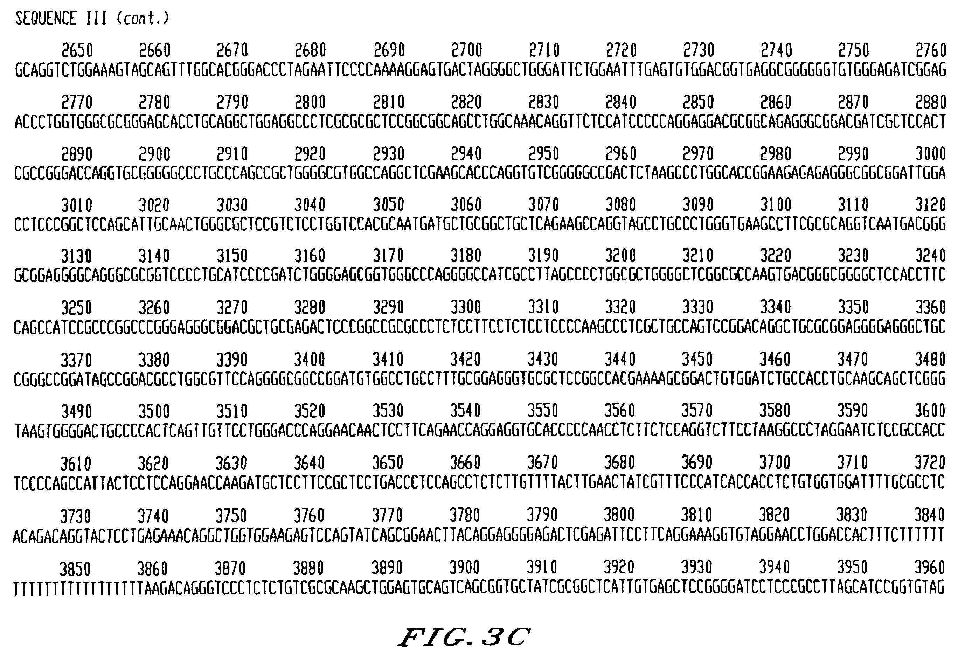

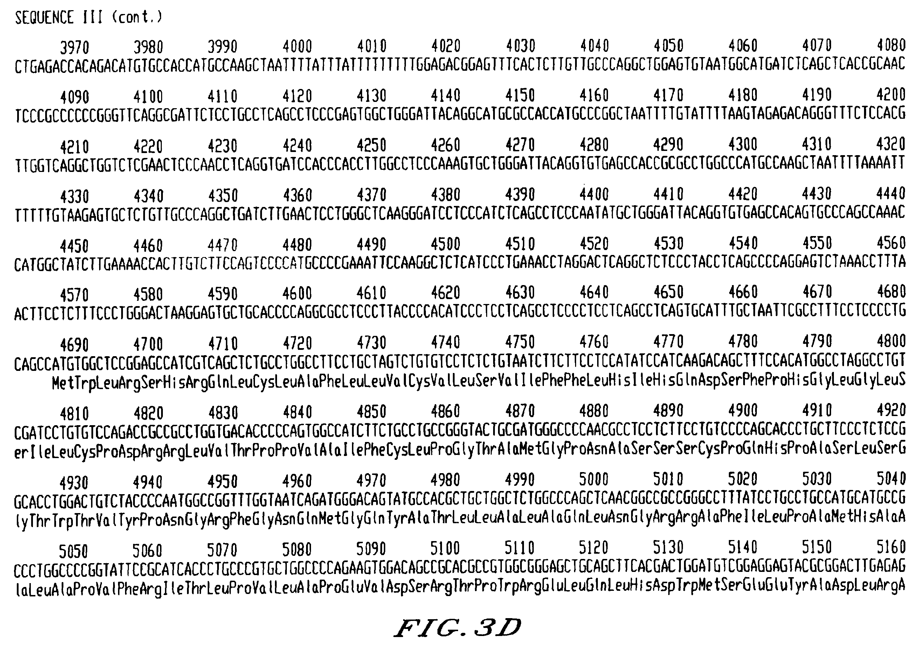

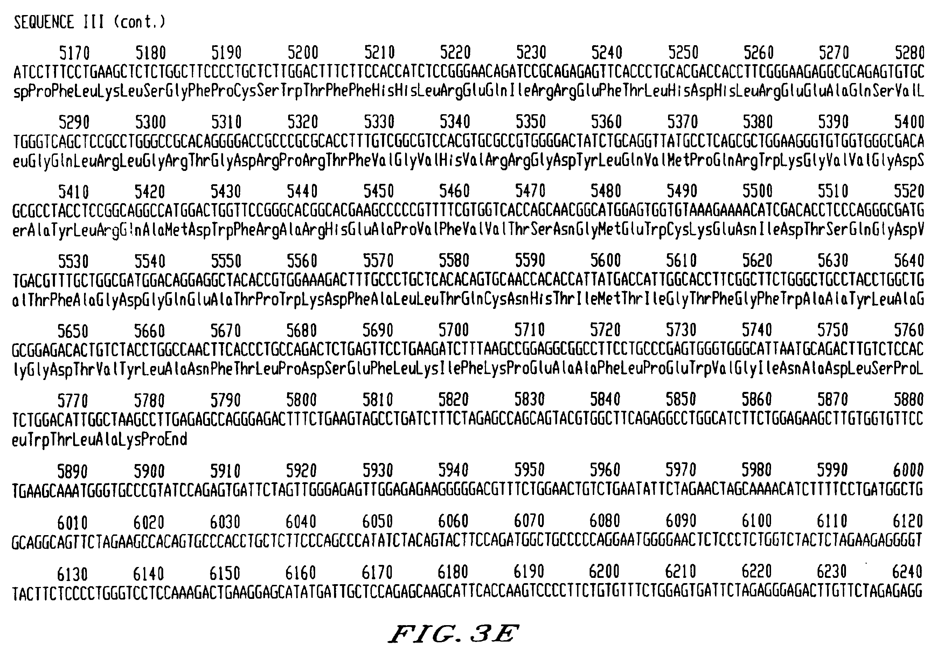

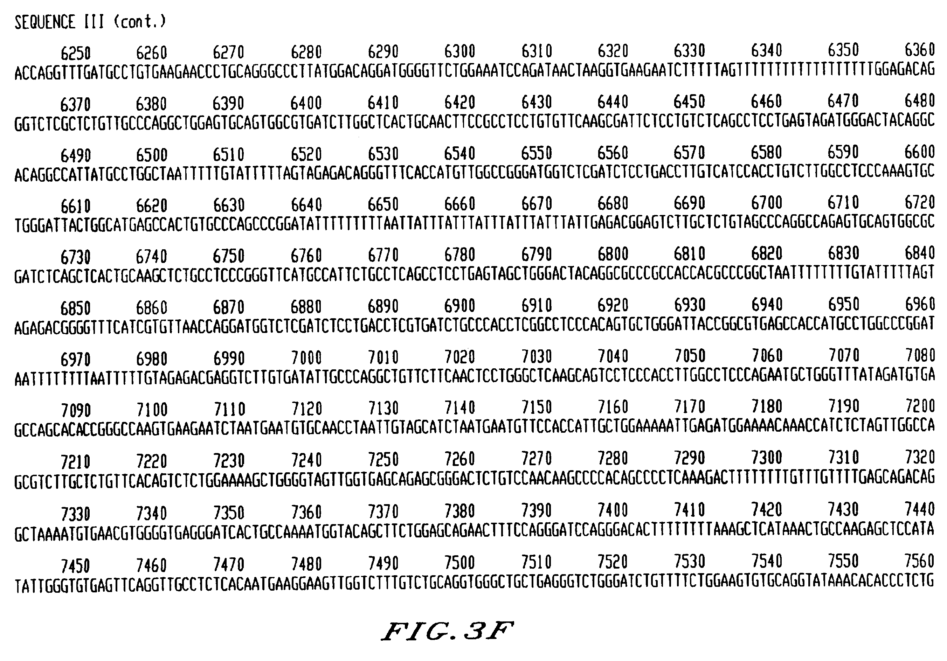

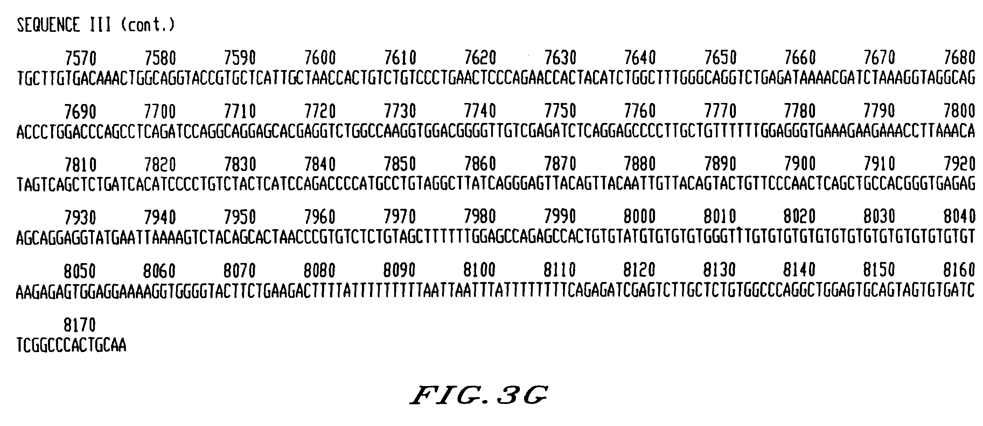

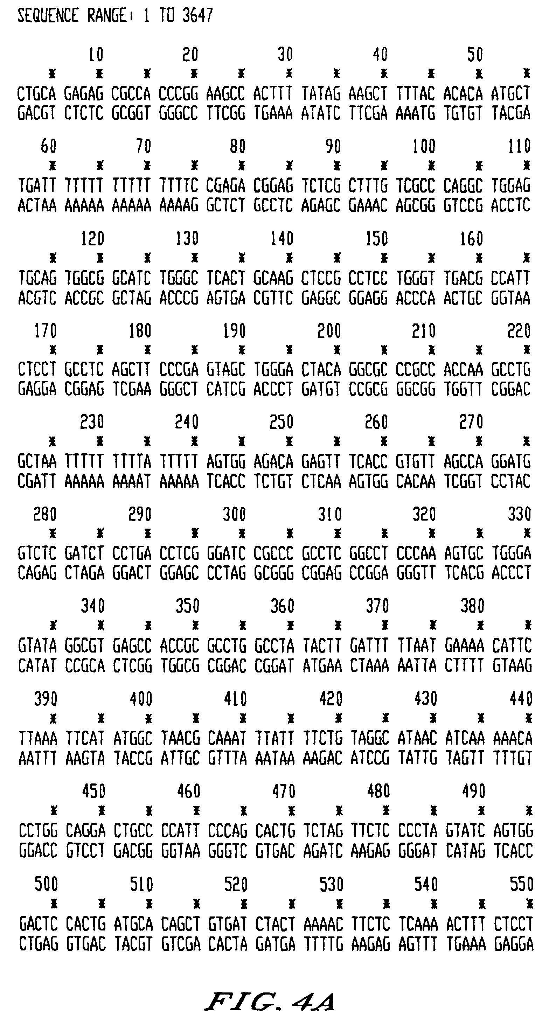

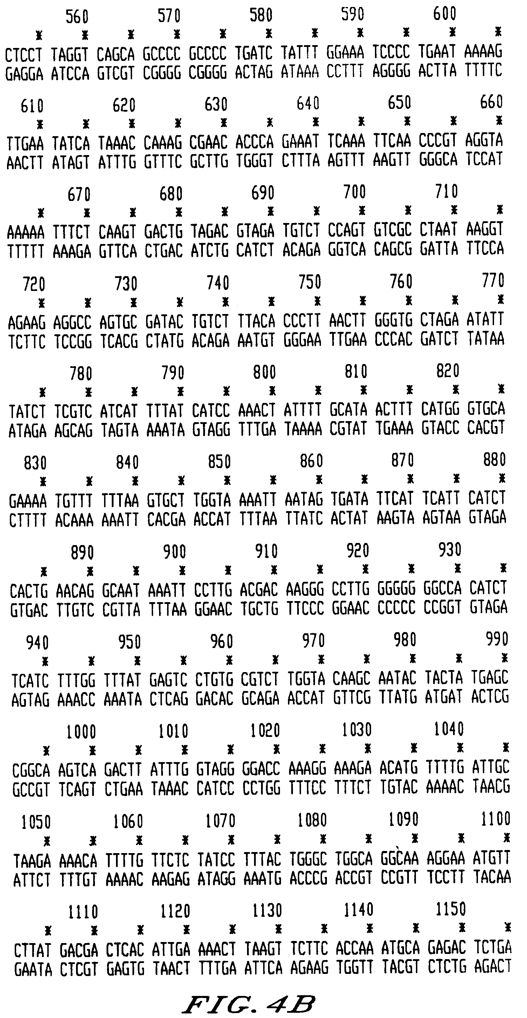

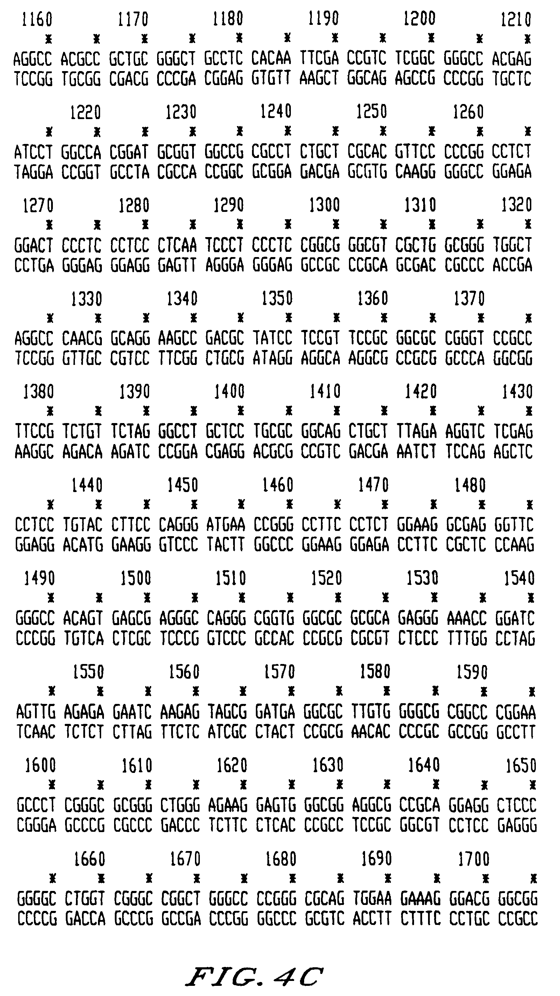

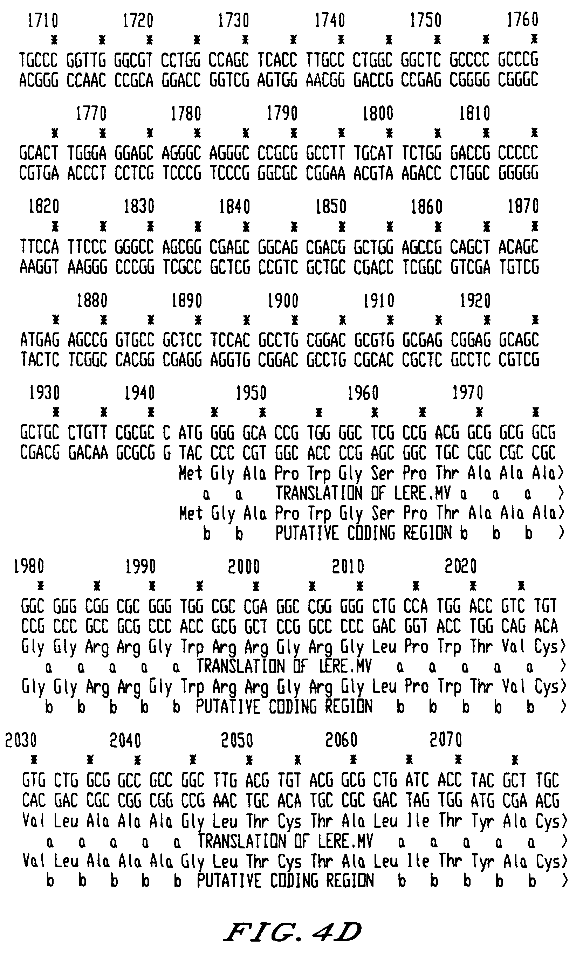

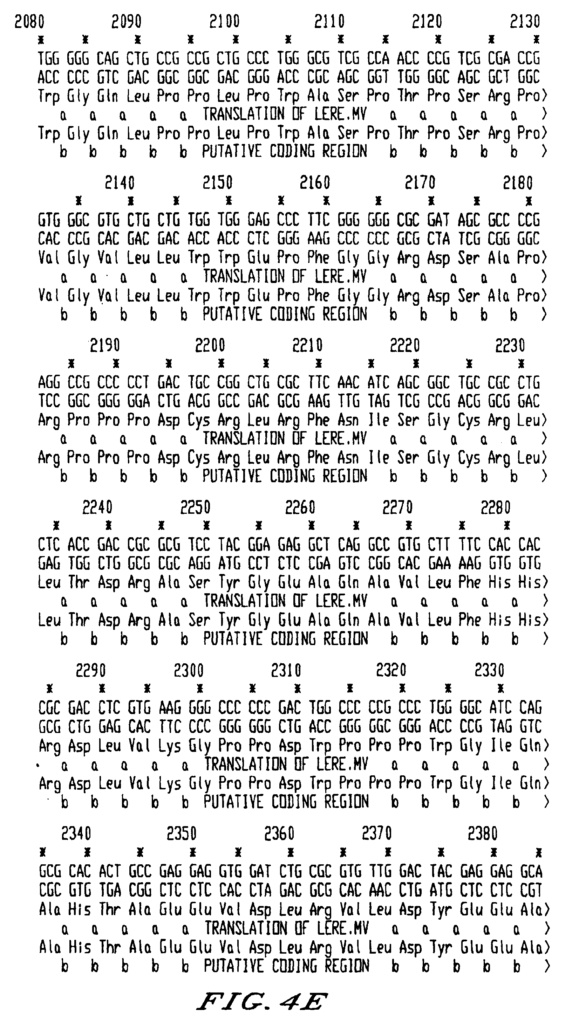

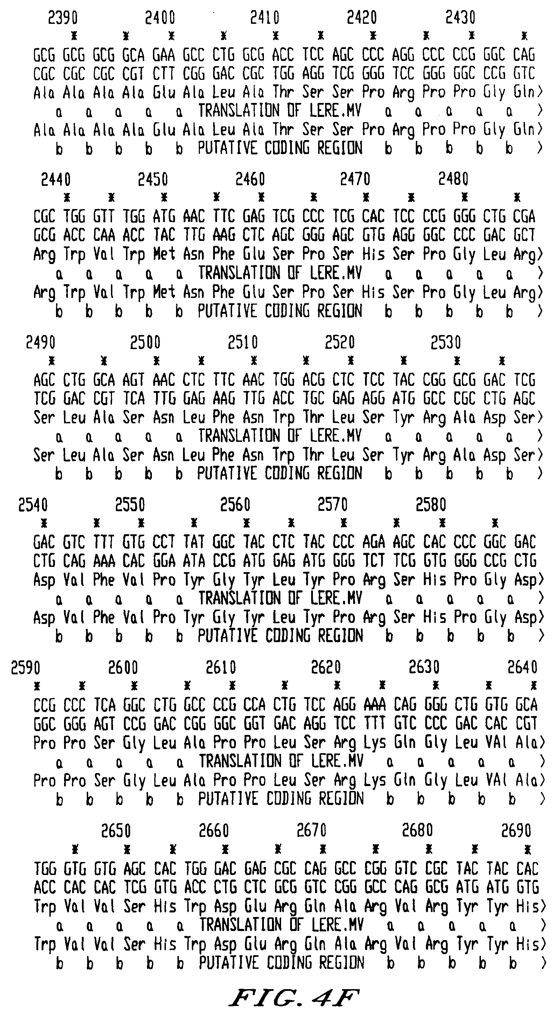

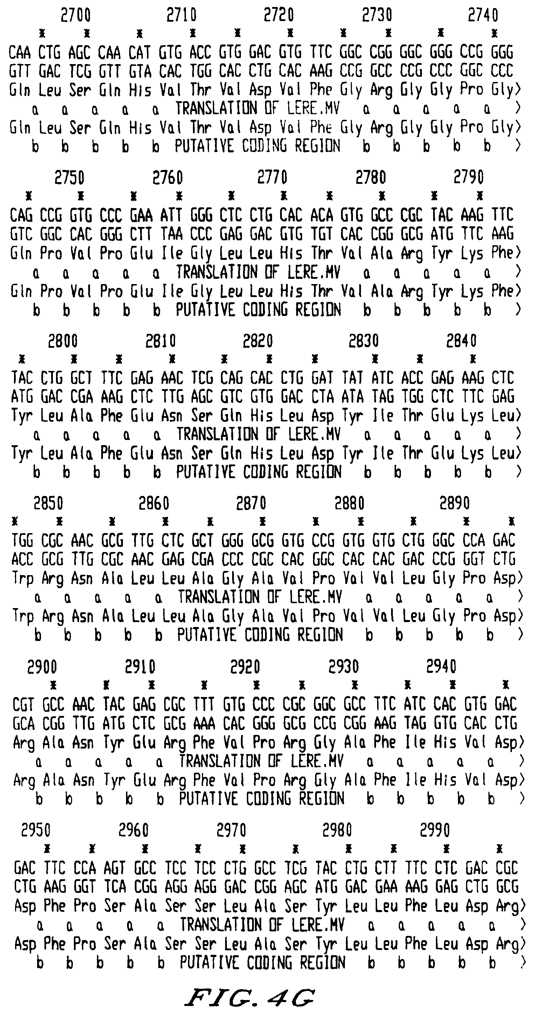

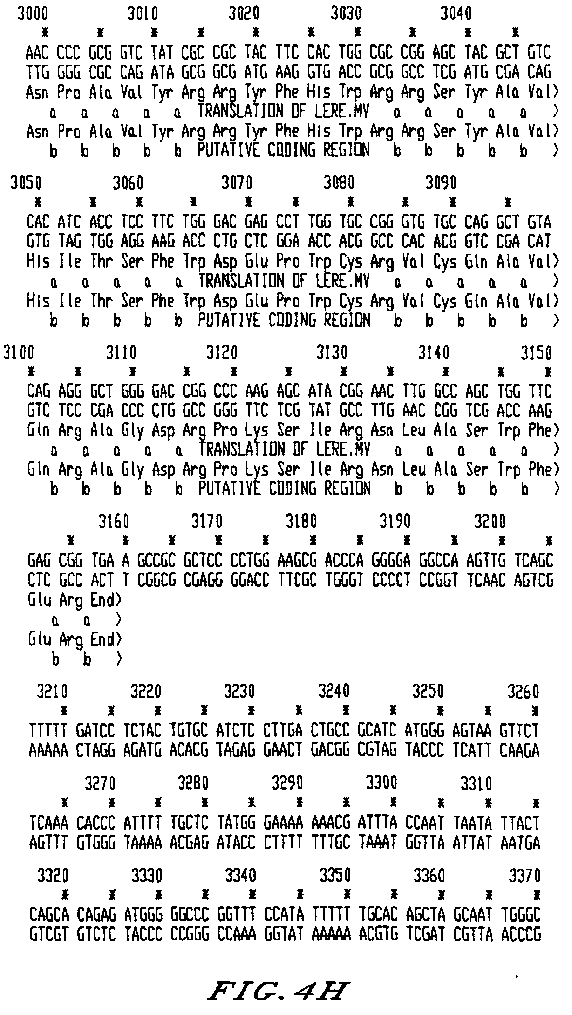

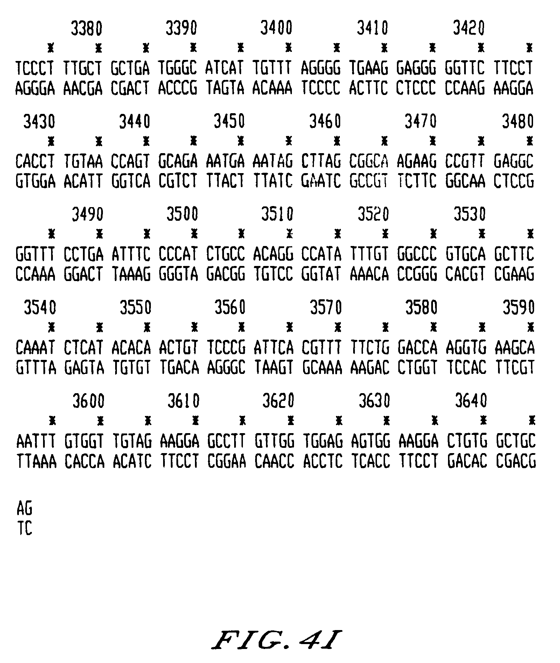

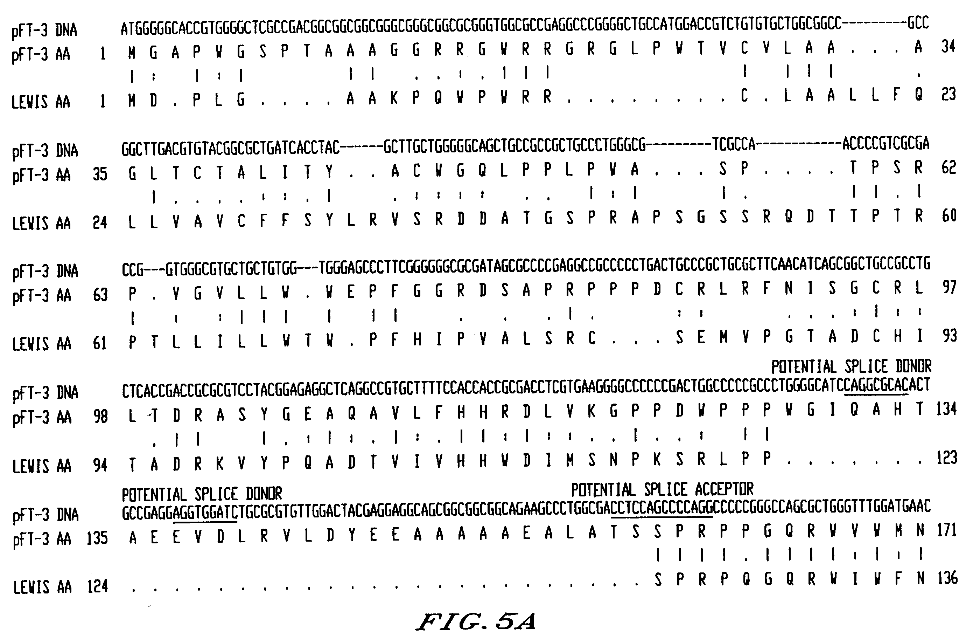

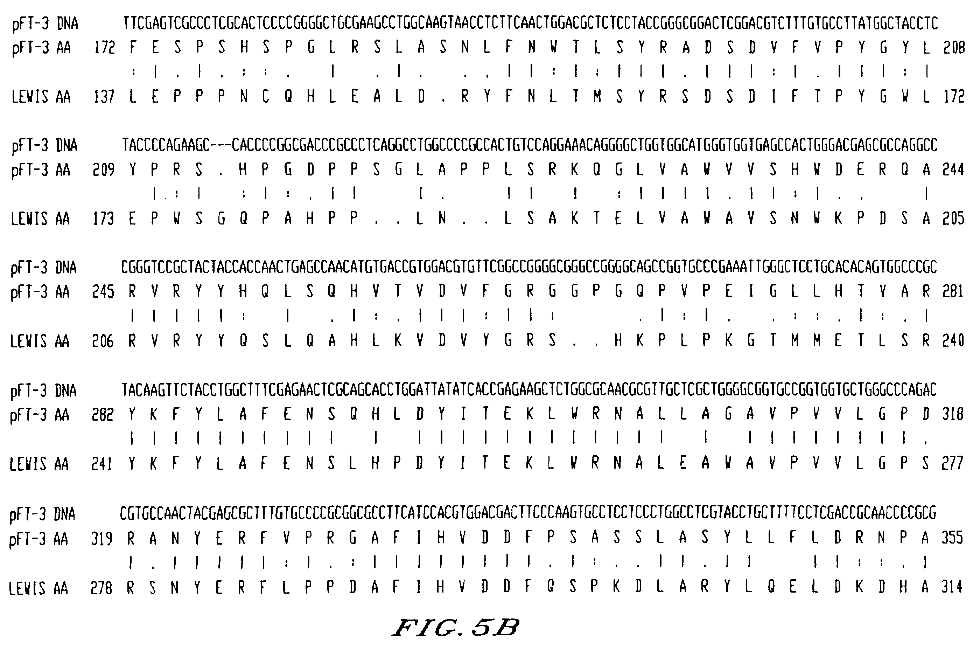

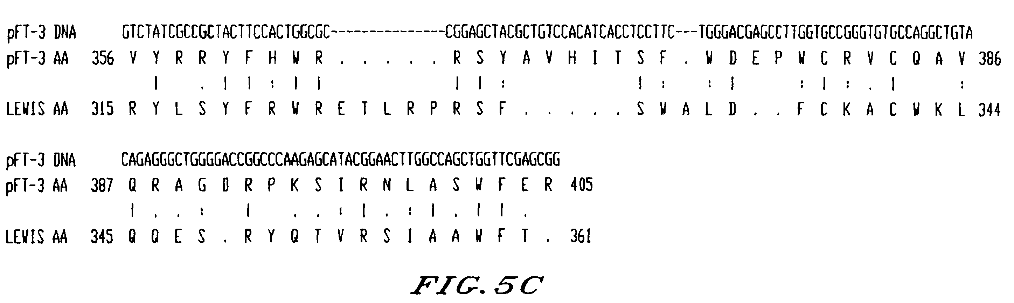

In another embodiment, the invention provides DNA sequence (IV) (set forth in Figure 2) that encodes a protein sequence capable of functioning as a GDP-Fuc:[β-D-Gal(1,4))-D-GlcNAc α(1,3)-Fucosyltransferase. This enzyme, when expressed by the cloned DNA sequence (IV) functions within mammalian cells to generate de novo expression of specific cell surface glycoconjugate structures of those cells.

The DNA sequence and corresponding peptide provided in this embodiment of the invention must correspond at least to the segment from nucleotide position 2089 to 3156, preferably positions 1942 to 3156, of Sequence (IV) set forth in Figure 2. These DNA sequences, can have further DNA sequences attached optionally to each end. These pendent DNA sequences can be of any length and up to a length corresponding to that set forth in Figure 2.

In a preferred embodiment, this embodiment of the invention provide DNA sequences, and their corresponding proteins, corresponding at least to the sequence between nucleotide positions 2089 to 3156, preferably positions 1942 to 3156, in Sequence (IV) and having attached to each end, optionally, further DNA sequences corresponding to those set forth in Figure 2. In this case, the pendent DNA sequences, and corresponding proteins, can be of any length and up to the length set forth in Figure 2.

These glycoconjugate structures, constructed in part by this enzyme, are recognized by an antibody against the stage specific embryonic antigen I (SSEA-1 or Lewis x; structure Galβ (1,4) (Fuc α (1,3)]GlcNAc), and by an antibody against the VIM-2 determinant NeuAcα (2,3)Galβ(1,4)GlcNAcβ--(1,3)Galβ(1,4)[Fucα(1,3)]GlcNAc. This enzyme, when expressed by DNA sequence (IV) functions in the enzymatic manner implied in its name, when assayed in extracts prepared from cells that express the DNA sequence.

The oligosaccharide products of this enzyme represents fucose linked in alpha 1,3 configuration to the GlcNac residue of a "type II" lactosamine acceptor. Throughout the remainder of this disclosure, these products will be referred to as subterminal α(1,3) fucose residues.

Construction of animal cell lines with specific capabilities with respect to post-translational modification of the oligosaccharides on cell-surface, intracellular, or secreted proteins or lipids by sub-terminal α(1,3) fucose residues that represent the products of this enzyme (for the production of diagnostics and therapeutics by the biotechnology industry) is possible.

The cloned DNA sequences of the invention can be introduced by standard technologies into a mammalian cell line that does not normally express the cognate enzyme or its product (sub-terminal α(1,3)fucose residues on oligosaccharides), and transcribed in that cell in the "sense" direction, to yield a cell line capable of expressing subterminal α(1,3) fucose residues on oligosaccharides on oligosaccharides on cell-surface, intracellular, or secreted proteins or lipids. Alternatively, these cloned DNA sequences may be introduced by standard technologies into a mammalian cell line that does express the cognate enzyme and its product (sub-terminal α(1,3) fucose resides), and transcribed in that cell in the "anti-sense" direction to yield a cell line incapable of expressing sub-terminal α(1,3) and fucose residues on cell-surface, intracellular, or secreted proteins or lipids.

In one embodiment, the endogenous GDP-Fuc: [β-D-Gal(1,4))D-GlcNAc α(1,3)-Fucosyltransferase gene(s), in a mammalian cell expressing the cognate enzyme(s), may be inactivated with DNA sequence (IV) by homologous recombination techniques, or by "anti-sense" oligonucleotide approaches based upon the DNA sequence described herein, or by dominant negative mutant fucosyltransferase sequences that inactivate endogenous GDPFuc: [β-D-Gal(1,4)]-D-GlcNAc α(1, 3)-Fucosyltransferase(s) and that may be derived via mutagenesis and genetic selection schemes, in conjunction with the sequence information in this disclosure.

This method can be used to construct animal cell lines that will be suitable host cells for the production of diagnostic or therapeutic materials whose usefulness or efficacy depends upon the specific post-translational modification determined by this cloned DNA sequence and its cognate enzyme. For example, it is known that the biological effectiveness of many therapeutic proteins or peptides, recombinant or otherwise, may depend critically upon the oligosaccharide structure(s) that are covalently attached to them. The structure of these oligosaccharides is primarily a function of the number and kind of glycosyltransferase enzymes that are found in the cell used to produce these therapeutic products. Animal cells and yeasts are competent to perform these glycosylation reactions; however, not all glycosyltransferase enzymes are produced by every animal cell or yeast, and therefore, some oligosaccharide structures (including sub-terminal α(1,3) fucose residues generated by the enzyme encoded by the DNA sequence described here) are not produced by them.

The converse is also true, namely, that producing cells may express a glycosyltransferase analogous to, or identical to, the GDP-Fuc: [β-D-Gal (1, 4) ]-D-GlcNAC α(1,3) - Fucosyltransferase encoded by the DNA sequence described here. It is likely that sub-terminal α(1,3) fucose residues may alter the bioactivity (for better or for worse) of natural or recombinant therapeutic or diagnostic agents (glycoproteins or glycolipids) produced by mammalian or other eukaryotic hosts. Eukaryotic host cells that the biotechnology industry uses to produce these recombinant agents may be altered with the DNA sequence information and related information described in this invention, to add sub-terminal α(1,3) fucose residues to the oligosaccharides on recombinant product by expressing all or part of the cloned sequences described here in the desired host. Alternatively, sub-terminal α(1,3) fucose residues may be eliminated from the product produced in these host cells by the use of transfected "anti-sense" vector constructs, recombination-based gene inactivation, "anti-sense" oligonucleotide approaches, or dominant negative mutant fucosyltransferases, outlined above.

The "old" methods used for this process include an empirical approach to identify a cell line that does or does not express this particular enzyme or an enzyme that functions in a similar or identical manner, for the production of the appropriately modified recombinant or natural product. This is not always optimal since cell lines with this particular post-translation modification capabilities may not exist naturally, or may not be especially suited to high level production of an appropriately modified product.

Alternatively, unwanted sub-terminal α(1,3) fucose residues present on a therapeutic material produced by an empirically identified animal cell line must be removed chemically or enzymatically, a process that say be costly or inefficient.

The advantages of using the cloned, functional DNA sequence described here in conjunction with the technologies outlined above, relative to these-older methods, include the ability to construct lines that specifically lack the capability to generate sub-terminal α(1,3) fucose residues on the oligosaccharides of glycoproteins and glycolipids; properly constructed, these cell lines will eliminate any need for chemical or enzymatic treatment of a therapeutic or diagnostic material to remove unwanted sub-terminal α(1,3) fucose residues. Moreover, in the event that sub-terminal α(1,3) fucose residues are found to be desirable for a particular diagnostic or therapeutic product produced by animal cells, cell lines may be engineered with the cloned DNA sequence described here to generate these residues.

In another embodiment, the invention makes possible isolation of reagents suitable for efficient enzymatic synthesis and production of oligosaccharides (in enzyme reactors, for example).

Oligosaccharides may have therapeutic utility as immunomodulatory reagents in the field of organ transplantation. In particular, soluble and solid-phase oligosaccharides may find use as therapeutic agents with which to block or ameliorate antibody-mediated organ transplant rejection in cases involving incompatibility due to differences in the major blood group antigen systems of the organ donor and the recipient, including the Lewis blood group system. Likewise, soluble oligosaccharides may find use as therapeutic agents that function by blocking attachment of bacterial, viral, or parasitic pathogens to glycoconjugate "receptors" found on the surface of the animal tissues that these pathogens invade. For example there is evidence that portions of the Lewis blood group oligosaccharide antigen (containing sub-terminal α(1,3) fucose residues) serve as "receptors" for some forms of uropathogenic bacteria. Moreover, glycoconjugates, including sub-terminal α(1,3) fucose residues, have been implicated in modulating adhesive events between cells and between cells and their environment during developmental and differentiation processes. Likewise, subterminal (1,3) fucose residues are critical portions of the oligosaccharide ligand for ELAM-1. These events included binding of spermatozoa to eggs, and the initial events that mediate attachment of fertilized ova to the uterine wall at the beginning of implantation. These observations suggest, for example, the possibility that contraceptive uses for (biologically "natural") oligosaccharide molecules might exist.

Currently, oligosaccharides containing sub-terminal α(1,3) fucose residues are produced by chemical synthesis (a procedure that is inefficient and costly) or by isolation from natural sources (using costly and inefficient procedures that often require the processing of large quantities of animal or plant material, and the purification of the desired oligosaccharide from other contaminating oligosaccharides). The invention described here provides a mechanism to synthesize abundant quantities of purified GDP-Fuc:[β-D-Gal(1,4)]-D-GlcNAc α(1,3)-fucosyltransferase. This could be used to construct an enzyme bioreactor (enzyme in solution or immobilized on a solid phase matrix, for example via the protein-A moiety fused to the catalytic domain of the enzyme, as described in previous manuscripts published by the inventor) capable of enzymatic synthesis of structures containing sub-terminal α(1,3) fucose residues.

This may be more efficient than approaches involving chemical synthesis of structures containing sub-terminal α(1,3) fucose residues or their purification from natural sources, for a variety of reasons. One, the only chemicals necessary would be the enzyme substrates; these are easily obtained or synthesized. Two, enzymatic synthesis of such structures will produce only the desired product and the nucleotide diphosphate product of substrate hydrolysis. This latter chemical is found as the natural by-products of these reactions in animal cells, is relatively non-toxic, and may be easily separated from the oligosaccharide synthetic product. By contrast, chemical synthetic procedures typically generate numerous products of side reactions which must be removed, and which may be toxic as well. Similarly, purification of oligosaccharides from natural sources requires the removal of other contaminating oligosaccharides present in the natural material. Three, enzymatic catalysis is extraordinarily efficient; nearly complete conversion of substrate to product might be achieved. By contrast, chemical synthesis of sub-terminal α(1,3) fucose residues on oligosaccharides is a multi-step process; yields at each step may be much less than 100%, and the cumulative efficiency of current chemical synthesis procedures does not approach the efficiency possible with enzymatic synthesis. Similarly, purification of oligosaccharides with sub-terminal α(1,3) fucose residues from natural materials can entail significant losses inherent to the purification procedures required to separate the desired oligosaccharide from contaminating, irrelevant oligosaccharides, with inefficient isolation of the desired oligosaccharide.

Although the GDP-Fuc: [β-D-Gal (1,4) ]-D-GlcNAc α(1, 3)-Fucosyltransferase encoded by the DNA sequence described here may be partially purified from animal tissues for synthetic use, these purifications are themselves inefficient, primarily because the enzyme is typically present in very low abundance. This invention provides two mechanisms that may provide for the abundant production of this enzyme. First, this may be done through the construction and selection of animal cells that produce relatively large quantities of the enzymes. Alternatively, this cloned nucleic acid sequences may then be used with standard recombinant DNA technologies to produce large quantities of, glycosyltransferases in yeasts or in prokaryotic hosts. Furthermore, the sequence encoding this enzyme may be modified via standard molecular cloning schemes or mutagenesis to yield a recombinant fucosyltransferase with novel properties that make it more desirable than the wild-type enzyme. For example, the modifications might be made to the enzyme that make it more stable, or more suitable for immobilization in a bioreactor.

In another embodiment, the invention makes possible isolation of reagents suitable for producing recombinant GDP-Fuc:[β-D-Gal(1,4))-D-GlcNAc α(1,3)-Fucosyltransferase to be used directly as a research reagent, or to be used to generate antibodies against the GDP-Fuc:(β-D-Gal(1,4))-D-GlcNAc α(1,3)-fucosyltransferase, for research applications.

This invention provides two mechanisms for producing large quantities of this enzyme (see ii. above - i.e. specially constructed animal cells, or via natural or synthetic genes encoding these enzymes) which may be used as a research tool with which to study the structures and functions of oligosaccharides and glycoproteins. Likewise, the enzyme produced by this method, or the nucleic acid sequence and derived protein sequence provided by this method, may be used to generate antibodies to this enzyme (via synthetic peptides). These antibodies might also be used as research reagents to study the biosynthesis and processing of these enzymes, and might be used as an aid in their purification for all the uses described in this disclosure.

In another embodiment, the invention makes possible obtaining, antibodies to glycosyltransferases as diagnostic reagents. Aberrant expression of GDP-Fuc:[β-D-Gal(1,4)]-D-GlcNAc α(1,3)-Fucosyltransferase has been associated with malignancy in humans, suggesting that this enzyme might serve as a tumor marker for early detection of malignancy involving a number of human tissues. Enzyme tumor markers have typically been assayed in body fluids by activity assays, which may be subject to non-specificity due to competing glycosyltransferase activity. These assays may also be insensitive since it is possible that inactive enzymes night be useful as tumor markers but would not be detected by enzyme activity assays.

This invention provides a mechanism for generating antibodies to this enzyme (monoclonal and polyclonal antibodies against synthetic peptides constructed from information derived from cloned DNA sequence encoding GDP-Fuc:[β-D-Gal(1,4)]-D-GlcNAc α(1,3)-fucosyltransferase, or against the recombinant enzyme produced by eukaryotic or prokaryotic hosts). Antibodies specific for this GDP-Fuc: [β-D-Gal (1,4) ]-D-GlcNAc α(1,3)-Fucosyltransferase so produced could be used to detect and quantitate this glycosyltransferase in body fluids, with specificity and sensitivity exceeding enzyme activity assays, and with the possibility of serving as a tumor marker for early detection of malignancy.

The DNA sequence of the invention may be used to provide recombinant enzyme for use in screening natural and synthetic compounds for fucosyltransferase inhibitors or inactivators. For example, a number of studies have noted an association between increased numbers of cell surface sub-terminal α(1,3) fucose residues on oligosaccharides of a cell and the ability of that cell to metastasize in a malignant fashion. If there is a causal relationship here, then it can be imagined that drugs that inhibit the enzyme encoded by the sequence in this disclosure might be active as anti-tumor agents.

The reagents described in this disclosure will be useful to drug companies for screening compounds for anti-fucosyltransferase activity, since the cloned sequence may be used with standard techniques to produce relatively large amounts of pure fucosyltransferase. This will aid in screening since the effects of potential inhibitors will be tested on a pure enzyme, without the confounding effects that may occur in whole cell extracts or with partially purified enzyme.

In another embodiment, the invention makes possible the engineering of glycosyltransferase substrate specificity to generate novel glycoconjugate structures on secreted or cell-associated glycoconjugates. This invention provides a reagent (a cloned GDP-Fuc:[β-D-Gal(1,4)]-D-GlcNAc α(1,3)-Fucosyltransferase gene segment), that, when used with appropriate mutagenesis and genetic selection schemes, may allow the generation of mutant GDP-Fuc:[β-D-Gal(1,4)]-D-GlcNAc α(1,3)-Fucosyltransferases that generate glycosidic linkages different from that generated by the wild-type enzyme. These novel linkages may or may not be naturally occurring, and could find utility as moieties that enhance bioactivity of the molecules to which they are attached.

Alternatively, mutagenesis and selection approaches may be used to generate mutant GDP-Fuc:[β-D-Gal(1,4))-D-GlcNAc α(1,3)-Fucosyltransferases that act in a dominant negative fashion. The dominant negative mutants so generated might be used to inactivate endogenous glycosyltransferase activities when the product(s) of such an enzyme are not desired. Mutant GDP-Fuc:[β-D-Gal(1,4)]-D-GlcNAc α(1,3)-Fucosyltransferases might also be generated, for example, that function as fucosidases that hydrolyze various sugar-linkages (fucose, mannose, or others) from oligosaccharides in vitro and in vivo.

In another embodiment, the invention makes possible genotyping individuals at the fucosyltransferase locus. Absence of a fucosyltransferase similar or identical to the one encoded by the DNA sequence detailed here has been associated with renal abnormalities in one family, and conceivably be responsible for the abnormalities in this family and others. Restriction fragment length polymorphisms within or linked to the gene corresponding to this cloned gene segment may be used to genotype individuals at this locus, for the purpose of genetic counseling. Likewise, the molecular basis for such abnormalities night be elucidated via the study of the gene segment described here, should it be causally-related to the pathogenesis of the abnormalities.

### Materials:

The term "L-cells" used throughout the text refers to the mouse Laprt⁻tk⁻ cell line.

Lactose, N-acetyllactosamine, 2'-fucosyllactose (Fucα(1,2) Galβ(1,4)Glc), UDP-GalNAc, phenyl-β-D-galactoside, and Ficoll 400 were obtained from Sigma. L-Fucose was from Pfanstiehl Labs (Waukegan, IL). UDP[l-³H]N-acetylgalactosamine (8.7 Ci/mmol) and D-[U-¹⁴C]mannose (239 mCi/mmol) were from DuPont-New England Nuclear. D-[2-³H]mannose (16.3 Ci/mmol), L-[6-³H]fucose (72 Ci/mmol), L-[1-¹⁴C]fucose (58.7 mCi/mmol), GDP[U-¹⁴C]β-L-fucose (268 mCi/mmol), and [α-³²p]dCTP (3000 Ci/mmol) were from Amersham Corp. Nonradioactive GDP-fucose was kindly provided by Dr. Eric Holmes (Seattle). FITC-ECA was obtained from E-Y Labs (San Mateo, CA). Plasmid pSV2-neo was obtained from Dr. David Chaplin (Washington University, St. Louis). Restriction enzymes (New England Biolabs or Boehringer Mannheim) were used according to the manufacturer's instructions.

### Antisera:

Monoclonal anti-H, anti-A, and anti-B antibodies (mouse IgM) were purchased from Chembiomed, Ltd. (Alberta, Canada). Monoclonal anti-H antibody BE2 was prepared from BE2 hybridoma cell culture supernatants (see below). Anti-mouse IgM and FITC-labeled antimouse IgM (both antigen-affinity purified, goat) were from Sigma.

### Cell Lines and Culture:

Mouse Laprt⁻tk⁻cells were obtained from Dr. David Chaplin. Human A431 cells were from Dr. Brian Whiteley and Dr. Luis Glaser (Washington University, St. Louis). BE2 cells were obtained from the American Type Culture Collection. Cells were grown in Dulbecco's modified Eagle's medium (GIBCO) supplemented with 10% fetal calf serum (Hyclone, Logan, UT). Transfected cells were grown in media containing G418 (GIBCO) at 400 µg/ml (active drug).

### Preparation of Genomic DNA:

High molecular weight genomic DNA was prepared from cultured cells by standard methods. Samples of genomic DNA were electrophoresed through 0.3% agarose gels buffered in Tris-acetate-EDTA to confirm their integrity and to estimate the average size of the molecules in the preparations.

### Transfections:

The calcium phosphate precipitation method was used to transfect mouse L cells with human genomic DNA. Cells (5 X 10⁵/100-mm dish) were incubated overnight with DNA precipitates 20-30 µg of genomic DNA and 1 µg of pSV2-neo). No exogenous pSV2-neo DNA was included in transfections that generated the mHs2 secondary library. The cells were fed fresh media the following day and were placed under G418 selection the next day. Transfection efficiencies were estimated by harvesting transfected cells 1 day after addition of DNA and plating duplicate serial dilutions of the cell suspensions. One set of dilutions was grown under G418 selection, and the other was grown in the absence of antibiotic to allow the derived transfection efficiencies to be corrected for plating efficiency. After 2 weeks of growth, colonies were counted after staining with 0.2% methylene blue in 50% methanol. Approximately 2000 independent tranfectants were typically obtained by transfecting 5 X 10⁵ cells on a 100-mm dish.

### Immunologic Selection of H-expressing Transfectants:

Transfectants were removed from culture dishes by incubating them with PBS containing 3 mM EDTA. Detached cells were washed and resuspended in staining medium (10 mM Hepes, pH 7.4, 0.1% sodium azide, 2% fetal calf serum in Dulbecco's modified Eagle's medium). The cells were kept at 4°C throughout the panning or cell sorting procedures. Cell cloning was done by plating cells at low density, allowing individual cells to form colonies, and isolating individual colonies with cloning cylinders.

Panning - Bacteriological culture dishes (Falcon 1007, 60 mm) were prepared for panning. Goat antimouse IgM was coupled to the dishes by incubating them overnight at 4°C with 4 ml of antibody solution diluted to 10 µg/ml in 50 mM Tris, pH 9.5. The antibody solution was aspirated, and the dishes were washed twice with PBS. The dishes were then blocked by incubating them at room temperature for at least 1 h with PBS containing 1 mg/ml bovine serum albumin. Dishes were then used immediately or were stored indefinitely at 4°C. The dishes were washed three times with PBS prior to use.

Cells to be panned were resuspended at a concentration of 10⁷/ml, in staining media containing anti-H antibody at 10 µg/ml. The cells were incubated for 30 min at 4°C, and unbound antibody was removed by pelleting the cells through 10 ml of PBS containing 1 mM EDTA, 0.1% sodium azide, and 2% Ficoll 400. After centrifugation, the supernatant was carefully aspirated, and the cells were resuspended at 10⁶/ml, in staining media. Three-ml aliquots of this cell suspension were applied to 60-mm panning dishes coated with goat anti-mouse IgM. The dishes were then incubated for 1 h at 4°C, and were then rinsed 5 times with serum-free Dulbecco's modified Eagle's medium to remove nonadherent cells. Fresh, serum-replete media was added to the dishes, and they were returned to the tissue culture incubator. The next day, adherent cells were removed with trypsin-EDTA and were replated on standard tissue culture dishes. These cells were grown for 10-18 days prior to the subsequent selection.

Cell Sorting - Transfectants were prepared for FACS analysis by incubating them for 30 min at 4°C with monoclonal IgM anti-H antibody (10 µg/ml in staining media) or with a control monoclonal IgM anti-B antibody (10 µg/ml in staining media). The cells were then washed in ice-cold staining media, and incubated for 30 min at 4°C in staining media containing fluorescein-conjugated goat antimouse IgM at 40 µg/ml. The cells were washed, resuspended in staining media, and subjected to analysis by the FACS (Coulter Electronics model Epics C). Samples were gated on forward and 900 light scatter to eliminate dead cells from analysis. H-expressing cells were collected aseptically into staining media and then returned to culture for 10-18 days before additional selections.

Rosette Procedure - A rosetting method was used to identify colonies of transfectants that bound anti-H antibody. This was done on 100-mm dishes containing isolated colonies comprised of approximately 100-300 cells. Plates of colonies were first rinsed twice with PBS and were then incubated for 1 h at 4°C with 4 ml of mouse IgM monoclonal anti-H antibody at 10 µg ml in PBS, 2% fetal calf serum, 0.1% sodium azide. The plates were then rinsed three times with PBS, and were incubated for 30 min at 4°C with 4 ml of human erythrocytes conjugated with goat anti-mouse IgM.
(Goat anti-mouse IgM was coupled to human blood group 0 red cells with chromic chloride. After conjugation, the red cells were washed with PBS, diluted to a 0.2% v/v suspension in PBS, 2% fetal calf serum, 0.1% sodium azide, and were used immediately.) Afterwards, the suspension of erythrocytes was carefully aspirated, and the plates were entirely rinsed with PBS and examined on a light box. Colonies that had bound anti-H antibody were macroscopically visible as "rosettes" consisting of red cells adherent to the colonies.

### Paper Chromatography:

Descending paper chromatography was performed using Whatman No. 3mm or Whatmann No. 1 in the following solvent systems: Solvent A, ethyl acetate/pyridine/water (10:4:3); Solvent B pyridine/water/ethyl acetate (10:11:5:36), upper phase. The dried chromatograms were cut into 1-cm strips and radiolabeled compounds were eluted with water. Radioactivity in an aliquot of each eluate was determined by scintillation counting.

### Analysis of L Cell GDP-Fucose Content:

To identify GDP-fucose in mouse L cells, cells (2.5 X 10⁶) were labeled for 3 days with 250 µCi of D-[2-³H]mannose in 30 ml of complete media. Cells were harvested and extracted with 60% ethanol for 5 min in a boiling water bath. The aqueous extract containing nucleotide sugars was concentrated under vacuum and resuspended in a small volume of water. Unlabeled GDP-mannose (270 nmol) and GDP-fucose (130 nmol) were added as internal standards, as the mixture was subjected to gel filtration chromatography on a Sephadex G-25 column (0.9 X 42-cm) equilibrated in 50 mM ammonium acetate. The eluate was monitored at 268 nm; fractions containing GDP-fucose and GDP-mannose were pooled, concentrated under vacuum, and resuspended in 200 ml of water. This was subjected to fractionation by HPLC on a weak anion exchange column (AX300, 4 mm X 24 cm, Pierce Chemical Co.). The sample was applied to the HPLC column equilibrated in 100 mM triethylamine acetate, pH 7.0, and was elated with a linear gradient from 100 to 300 mM triethylamine acetate, pH 7.0 in 50 min at a flow rate of 2 ml/min. The eluant was monitored at 268 nm, and fractions (0.5 ml) were collected for scintillation counting and subsequent analysis. The unlabeled nucleotide sugar internal standards were identified by their characteristic elation times (GDP-fucose, 35.5 min. approximately 800 cpms; GDP-mannose, 33.0 min. approximately 1000 cpms). The radioactive peaks corresponding to the fractions coeluting with unlabeled GDP-fucose and GDP-mannose were subjected to hydrolysis with 0.1 N HC1 for 45 min at 100°C. These were then fractionated by descending paper chromatography on Whatman No. 3MM in solvent B for 20 h, in parallel with L-[¹⁴C]fucose and D-[¹⁴C] mannose standards. In each case, approximately 30% of the counts hydrolyzed were recovered as the appropriate monosaccharide.

### Analysis of Fucose-labeled Glycopeptides:

Radiolabeled glycopeptides were prepared and analyzed. Mouse L cells (2 X 10⁶) were labeled for 3 days with 200 µCi of L-[6-³H]fucose in 20 ml of complete media. Cells were harvested and extracted with chloroform, and then water, and the pellet remaining after the final extraction was subjected to exhaustive digestion with Pronase (Behring Diagnostics). This was then desalted by gel filtration chromatography on Sephadex G-25-80. This material was concentrated under vacuum, and an aliquot was hydrolyzed in 0.1 N HCl at 100°C for 45 min. The hydrolysate was then subjected to descending paper chromatography on Whatman No. 3mm in Solvent B for 20 h, in parallel with an L-[¹⁴C]fucose standard. Approximately 26% of the counts present in the macromolecular material were released and cochromatographed with the radiolabelled fucose standard.

### Indirect Immunofluorescence:

Immunofluoroscence was performed on cells plated on 8-well tissue culture chamber slides (Lab-Tek). Cells were plated at a density of 5 X 10⁴/well 24 h prior to analysis. Anti-H and anti-A primary antibodies were diluted in PBS containing bovine serum albumin at 2 mg/ml, to a final concentration of 10 µg/ml. Cell-bound primary antibodies were detected with FITC-conjugated goat anti-mouse IgM, diluted to 40 µg/ml in PBS containing 2 mg/ml bovine serum albumin.

Hapten Inhibition - Plated cells were washed twice with PBS, and were incubated for 30 min at 4°C with 100 µl of diluted anti-H antibody, or with 100 µl of diluted antibody containing different oligosaccharide haptens, each at a concentration of 20 mM. The chambers were then washed twice, and 100 µl of FITC-conjugated goat anti-mouse IgM was added. After 30 min at 4°C, the chambers were washed three times with PBS, and the cells were fixed at room temperature for 10 mm in 3.7% formaldehyde in PBS. The cells were washed twice with PBS, the chambers were removed, and the slide was mounted in PBS containing 25% glycerol. Cells were examined by fluorescence microscopy using a Zeiss Axiophot photomicroscope equipped with fluorescence epiillumination.

### Southern Blotting:

Genomic DNA was digested to completion with restriction enzymes (8 units µg DNA, overnight digestion). The restriction fragments were fractionated by electrophoresis through 0.6% agarose gels buffered in Tris-borate-EDTA. The DNA fragments were then transferred to nylon membranes (Hybond-N, Amersham Corp. , according to the standard Southern blotting method. Blots were prehybridized at 39°C for at least 2 h in 50% formamide, 5 SSC (1 X SSC is 150 mM NaCl, 15 mM sodium citrate, pH 7.0, 1 X PE is 50 mM Tris, pH 7.5, 0.1% sodium pyrophosphate, 1% sodium dodecyl sulfate, 0.2% polyvinylpyrolidone (Mᵣ4O,OOO), 0.2% Ficoll (Mᵣ4OO,OOO), and 5 mM EDTA), and 150 µg/ml denatured salmon sperm DNA. Hybridizations were done in the same solution at 39°C for at least 16 h. Blots were washed four times at room temperature in 2 X SSC, 0.1% sodium dodecyl sulfate, and then once for 30 min at 65°C in 0.75 X SSC, 0.5% sodium dodecyl sulfate. The BLURS probe consisted of a 300-base pair BamHI segment isolated from the BLUR8 plasmid. This fragment was subjected to two cycles of gel purification prior to labeling to ensure that it was free from contaminating plasmid sequences. Probes were labeled with [α³²p]dCTP to a specific activity of at least 5 X 10⁸ cpm µg using the random priming method.

### Isolation of a cloned human cDNA that encodes a GDP-Fuc:[β-D-Gal(1,4/1,3)]-D-GlcNAc(/Glc)α(1.3/1.4)-fucosyltransferase.

In one embodiment, the present invention provides a gene transfer system that allows isolating cloned cDNAs encoding functional α(1,3)fucosyl-transferase or α(1,4)fucosyltransferase [α(1,4)FT] molecules or that otherwise determine α(1,3)FT or α(1,4)FT expression, without the need to first purify the enzyme. This system instead exploits existing reagents that allow detection of the cell surface-expressed oligosaccharide product of these enzymes, and that provide for specific assay of their enzymatic activity.

This approach requires a recipient host cell with specific properties that allow selection of the appropriate cloned cDNA molecules. The host must not express α(1,3)FTs, nor cognate surface Gal β(1,4)[Fucα(1,3)]GlcNAc linkages (SSEA-1 structures). However, this host must synthesize the appropriate substrates for surface display of SSEA-1 molecules. These substrates include the nucleotide sugar GDP-fucose, and surface-expressed glycoconjugate molecules that may serve as oligosaccharide acceptors for the transglycosylation reaction. Each of these properties are fulfilled by COS-1 cells.

Fluorescence-activated cell sorter (FACS) analysis indicated that COS-1 cells do not express surface-localized SSEA-1 determinants. Enzyme analyses performed with COS-1 extracts confirmed that absence of SSEA-1 expression was due to a deficiency of α(1,3)FT activity. The inventor expected that COS-1 cells would contain substrate levels of GDP-fucose within their Golgi, since with the exception of certain lectin-resistant mutant cells lines, virtually all mammalian cells synthesize GDP-fucose and translocate it into the Golgi lumen. COS-1 cells also construct surface-expressed glycoconjugates containing unsubstituted polylactosamine moieties that represent potential oligosaccharide acceptors for α(1,3)FT activity determined by a transfected cDNA. The inventor confirmed that these substrates are available for use in the construction of surface-expressed, terminal fucose linkages by demonstrating expression of a different terminally-linked fucose linkage (H Fucα(1,2)Gal) on COS-1 cells after transfection with a cloned human gene fragment that the inventor had previously shown to determine expression of an α(1,2)FT. The inventor therefore observed that COS-1 cells could construct surface-expressed SSEA-1 molecules following transfection with α(1,3)FT-determining cDNAs.

### Isolation of a Cloned CDNA That Determines Expression of an α(1,3)FT and Surface-Localized SSEA-1 Structures

The human A431 cell line has been shown to express cell surface Lewis blood group a and b structures that represent the products of an α(1,4)FT. Enzyme assays performed with A431 extracts confirmed that cells also express a corresponding α(1,3)FT activity. A cDNA library was therefore constructed with A431 cell mRNA in the mammalian expression vector pCDM7 and was transfected into COS-1 cells. The transfected cells were screened by the procedure of Seed using a monoclonal antibody specific for SSEA-1 determinants.

In order to follow enrichment for an α(1,3)FT-determining cDNA during the selection procedure, an assay was employed in which 2'-fucosyllactose was used as an acceptor substrate. This acceptor can discriminate between the Lewis α(1,3/1,4)FT and non-allelic human α(1,3)FTs, since it is used efficiently by the former enzyme but not by the latter. With this assay, the inventor was unable to detect any enzyme activity in COS-1 cells transfected with the A431 cDNA library, or in cells transfected with amplified plasmid DNA isloated from the initial selection. However, amplified plasmid DNA obtained from the second selection was found to direct a low level of enzyme activity when transfected into COS-1 cells. A modest increment in enzyme activity was obtained after a third selection by panning. At this stage, "sib selection" was employed to identify and isolate a cloned α(1,3)FT cDNA. Pools of clones isolated from the third panning selection were tested for their ability to generate α(1,3)FT activity in transfected COS-1 cells. From these experiments, it was estimated that approximately one in 500 clones represented a plasmid that determined α(1,3)FT expression. One "active" pool of approximately 400 clones was further subdivided and the resulting pools were tested for their ability to generate enzyme activity in transfected cells. One clone (pCDM7-α(1,3/1,4)FT) in an active 16 clone pool was found to direct very high level expression of the α(1,3)FT. FACS analysis was used to confirm that this plasmid also directs surface expression of SSEA-1 determinants. COS-1 cells transfected with this plasmid stain brightly with anti-SSEA-1 antibody, but not with a control IgM anti-H antibody, whereas cells transfected with pCDM7 vector alone exhibit background staining with both antibodies.

### Deduced Protein Sequence of the Cloned cDNA Predicts a Transmembrane Topology

The cDNA insert in pCDM7-α(1,3/1,4)FT (Sequence I) is 2022 nucleotides in length, and consists of a 72 bp 5' untranslated region, a continuous open reading frame of 1083 bp, and a 3' untranslated region of 867 bp that is terminated by a poly(A) tail.

This cloned cDNA hybridizes to a single prominent 2.3 kb transcript in A431 cells showing that this insert is essentially full-length. The nature of an additional faint 7.5 kb transcript is at present undefined. The initiation codon at the beginning of the long open reading frame is embedded within a sequence similar to the Kozak consensus initiation sequence and is preceded by two in-frame stop codons. There is also a single additional ATG upstream from the assigned initiator. This ATG also fits the Kozak consensus sequence, but initiates a very short in-frame sequence. The long open reading frame predicts a 361 amino acid protein of Mr 42,069 Da. Sequence comparisons with the latest DNA and protein sequence databases (Protein Identification Resource, Release 21.0, and GenBank, Release 60.0) identified no sequences with significant similarity to this sequence, except for a segment within the 31 untranslated region that is similar to human Alu sequences. The 3' untranslated region also contains 20 degenerate copies of a 16 nucleotide sequence of unknown functional significance.

Comparions between the sequence predicted by the insert in pCDM7-α(1,3/1,4)FT and four different cloned mammalian glycosyltransferases revealed no obvious primary sequence similarities. While these latter enzymes also share no extensive primary sequence similarities, they exhibit an analogous overall structural organization. Specifically, these enzymes are representative of Type II transmembrane proteins, each composed of a short, NH₂-terminal cytoplasmic domain, a single transmembrane segment, and a larger, COOH-terminal catalytic domain that ultimately inhabits the Golgi lumen. Inspection and hydropathy analysis of the predicted protein sequence suggested that this protein maintains a similar structural organization. There is a single hydrophobic segment near the amino terminus that is comprised of 19 amino acids and is flanked by basic residues. This putative signal-anchor sequence would place 327 amino acids within the Golgi lumen, while leaving 15 residues within the cytosolic compartment.

### The Protein Encoded by pCDM7-α(1,3/1,4)FT is a Fucosyltransferase

Expression data and the predicted topological similarity of this sequence to other glycosyltransferases, show that this cDNA encodes an α(1,3)FT. Nonetheless, these data are also formally consistent with the possibility that this cDNA sequence instead encodes a molecule that trans-determines α(1,3)FT activity by interaction with an endogenous gene, transcript, or protein. To demonstrate that enzymatic activity is directly associated with this molecule, the putative catalytic domain of the predicted polypeptide was fused to a secreted form of the IgG binding domain of Staphylococcus aureus protein A in the mammalian expression vector pPROTA, to generate the vector pPROTA-α(1,3/1,4-Ft)_{c}. Since this fusion protein would lack the putative transmembrane anchoring segment of the fucosyltransferase, we expected it would be synthesized as a secreted molecule that could be afinity-purified on an IgG-containing matrix and subsequently tested for α(1,3)FT activity. COS-1 cells transfected with the control vectors pCDM7 or PROTA generated no detectable cell-associated or released enzyme activity. However, conditioned media prepared from COS-1 cells transfected with pPROTA-α(1,3/1,4)FT_{c} or with pCDM7-α(1,3/1,4)FT, contained significant quantities of α(1,3)FT activity. Significantly, virtually 100% of the released activity generated by pPROTA-α(1,3/1,4)FT_{c} was specifically retained by the IgG-Sepharose matrix, and approximately 24% of this activity could be recovered from the matrix after exhaustive washing. By contrast, the released activity generated by pCDM7-α(1,3/1,4)FT did not interact with the affinity adsorbent. These results indicate that the protein encoded by this cloned cDNA encodes a fucosyltransferase, demonstrate that information sufficient to generate α(1,3)FT activity resides within the enzyme's COOH-terminal 319 amino acids, and show that this approach can be used to affinity purify the catalytic domain in an enzymatically active state as a portion of a bipartite fusion protein.

### The Fucosyltransferase is a Glycosylated Transmembrane Protein

In order to confirm the transmembrane topology predicted for the enzyme, fucosyltransferase cRNA was prepared and was subjected to analyses by a series of in vitro translation experiments. The ³⁵S-methionine-labelled primary translation product generated in these experiments migrated with a molecular weight of approximately 37,500 Da. The discrepancy between this observed molecular weight and the predicted one (42,069 DA) may be reconciled by the observation that membrane-spanning proteins often migrate in an anomalously rapid manner through SDS-polyacrylamide gels, relative to soluble protein molecular weight markers. When this radiolabelled protein was generated by in vitro translation in the presence of canine pancreatic microsomes, it migrated with an Mr of approximately 42,000 Da. The ∼ 6,000 Da increase in molecular mass observed when the translations were performed in the presence of microsomes suggests that two core glycosylation structures are added by microsomal oligosaccharyltransferase to the two potential asparagine-linked glycosylation sites during co-translational translocation across the microsomal membrane. This product also co-sedimented with the microsomes, suggesting that the protein had become co-translationally inserted within, or translocated across, the microsomal membrane. When this raiolabelled, microsome-associated protein was subjected to limit digestion with endoglycosidase H, its molecular mass was reduced to a one essentially identical to that of the primary translation product. Partial endoglycosidase H digestion generated an additional band or intermediate size, that likely consists of molecules that contain a single residual core glycosylation unit. These results indicate that the addition of core oligosaccharide structures is responsible for the increase in size of the protein observed in the co-translation experiments. These observations indicate that the two potential N-glycosylation sites found within the predicted fucosyltranserase amino acid sequence are glycosylated during translocation across the microsomal membrane.

Additional support for the predicted transmembrane topology of the fucosyltransferase was provided by the results of protease protection experiments. Co-translation in the presence of microsomes yields a 42,000 Da polypeptide that is resistant to digestion with protease. The protease-digested product migrates slightly faster than the undigested, microsome-associated polypeptide; this difference is most likely accounted for by removal of some or all of the 15 NH₂-terminal amino acids predicted to be displayed on the exterior of the microsomes. Addition of microsomes after translation yielded a protease-sensitive, non-glycosylated radiolabelled product, indicating that membrane insertion of the protein is a co-translational, but not post-translation, event. A small amount of a ∼ 34KDa polypetide that is protease-sensitive and glycosylated can also be identified in these experiments. The precise nature of this protein is unknown, but it appears in a proteinase K concentration-dependent manner. We therefore suspect that it represents a proteolytic fragment of the intact protein generated when the integrity of some microsomal vesicles is disrupted. In aggregate, these experiments indicate that the bulk of this polypeptide can be sequestered within the microsomal lumen by a co-translational translocation process, ultimately yielding a product that is N-glycosylated. These results are consistent with the type II transmembrane topology predicted by the fucosyltransferase sequence.

### The Fucosyltransferase Can Construct Two Distinct Glycosidic Linkages

It is believed that, in general, each glycosyltransferase is competent to perform a single transglycosylation reaction, that in turn generates a single glycosidic linkage. However, genetic and biochemical studies indicate that the human Lewis blood group locus may determine expression of a single fucosyltransferase capable of generating subterminal Fucα(1,3) and Fucα(1,4) linkages on several type I and type II acceptor substrates. In particular, the Lewis enzyme is thought to be the only human fucosyltransferase capable of using the acceptor 21-fucosyllactose. Since plasmid pCDM7-α(1,3/1,4) FT was isolated with an enrichment scheme involving an enzymatic assay based upon this acceptor substrate, it therefore seemed likely that its cDNA insert encodes the Lewis enzyme. To confirm this, the inventor performed a series of analyses to determine the acceptor specificities of the recombinant enzyme.

Extracts of COS-1 cells transfected with pCDM7-α(1,3/1,4)FT were tested for their ability to catalyze transglycosylations between GDP-[¹⁴C]fucose and the type I acceptor lacto-N-biose I, or the type II acceptors lactose and N-acetyllactosamine. There are only two possible classes of monofucosylated products that can be formed from each of these acceptors by known human fucosytransferases. These are H-active products containing a Fucα(1,2) linkage on the terminal Gal of these molecules, or Lewis x- or Lewis a-active products containing fucose linked in alpha anomeric configuration to the subterminal monosaccharide of these acceptors via either the monosaccharide's C4 hydroxyl (type I acceptor) or its C3 hydroxyl (type II acceptors). The inventor therefore fractionated the reaction products with a descending paper chromatography method that could distinguish between the two classes of reaction products possible with each acceptor, and thus allow determination of enzyme specificity.

The inventor found that lactose was utilized by the recombinant enzyme to form a radiolabelled compound with the chromatographic mobility characteristic of the Lewis x trisaccharide 3-fucosyllactose, and distinct from the other possible product, the type II H trisaccharide. Likewise, these extracts also generated 3-fucosyl-N-acetyllactosamine when N-acetyllactosamine was used as an acceptor. Radiolabelled fucose was quantitatively released from each product upon digestion with α-fucosidase, indicating that the enzyme attaches fucose to these acceptor substrates in alpha anomeric configuration. These results are consistent with the flow cytometry observations indicating that pCDM7-α(1,3/1,4)FT determines expression of the Galβ(1,4)[Fucα1,3]GlcNAc linkage representing the SSEA1-1 determinant.

Moreover, the radiolabelled product of the type I acceptor lacto-N-biose I chromatographed with a mobility distinct from the H active standard 2'-fucosyllacto-N-biose I and consistent with its identity as the Lewis a trisaccharide 4-fucosyllacto-N-biose I. This product was also susceptible to digestion with α-fucosidase. Identical results were obtained for all three disaccharide acceptors when affinity-purified protein A-fucosyltransferase was used in these experiments. Taken together, these results indicate that the recombinant fucosyltransferase can construct both Fucα(1,3) and Fucα(1,4) glycosidic linkages on type II and type I disaccharide acceptors, respectively.

In a complementary set of analyses, type I and type II blood group H trisaccharides were tested as acceptors for the enzyme encoded by the fucosyltransferase cDNA. Radiolabelled type I and type II H molecules were prepared by fucosylating their disaccharide precursors at the C2 hydroxyl of their terminal galactose residues, using cell extracts containing the blood group H α(1,2)-FT and GDP-·[¹⁴C]fucose. These HPLC-purified radiolabelled type I and type II H acceptors were then each used in reactions containing unlabelled GDP-fucose and affinity-purified fucosyltransferase activity generated by pPROTA-α(1,3/1,4)FTC. HPLC analysis of these reactions identified new radiolabelled compounds with chromatographic mobilities predicted for the Lewis b tetrasaccharide and the Lewis y tetrasaccharide, generated with the type I or type II acceptors, respectively. Virtually identical results were obtained with extracts prepared from COS-1 cells transfected with pCDM7-α(1,3/1,4)FT. In a third series of experiments, the inventor demonstrated that this enzyme can operate on "type I" or "type II" acceptors whose terminal galactose residues are substituted with sialic acid in α(2,3) linkage, to generate the sialyl Lewis x and sialyl Lewis a tetrasaccharide determinants. Flow cytometry analysis of COS-1 cells transfected with pCDM7-α(1,3/1,4) FT and stained with a monoclonal anti-sialyl Lewis x antibody indicates that this plasmid can determine surface expression of the sialyl Lewis x antigen, that is the product of α(1,3)FT action on "type II" acceptors whose terminal galactose residues are substituted with sialic acid in α(2,3) linkage. Likewise, COS-1 cells transfected with pCDM7-α(1,3/1,4)FT and stained with a monoclonal anti-sialyl Lewis a antibody indicates that this plasmid can determine surface expression of the sialyl Lewis a antigen, that is the product of α(1,4)FT action on type I acceptors whose terminal galactose residues are substituted with sialic acid in α(2,3) linkage.

In a third series of experiments, the inventor demonstrated that this enzyme can operate on type I or type II acceptors whose terminal galactose residues are substituted with sialic acid in α(2,3) linkage, to generate the sialyl Lewis x and sialyl Lewis a tetrasaccharide determinants. Flow cytometry analysis of COS-1 cells transfected with pCDM7-α(1,3/1,4)FT and stained with a monoclonal anti-sialyl Lewis x antibody indicates that this plasmid can determine surface expression of the sialyl Lewis x antigen, that is the product of α(2,3)FT action on type II acceptors whose terminal galactose residues are substituted with sialic acid in α(2,3) linkage. Likewise, COS-1 cells transfected with pCDM7-α(1,3/1,4)FT and stained with a monoclonal anti-sialyl Lewis a antibody indicates that this plasmid can determine surface expression of the sialyl Lewis aantigen, that is the product of α(1,4)FT action on type I acceptors whose terminal galactose residues are substituted with sialic acid in α(2,3) linkage.

These analyses indicate that the fucosyltransferase encoded by pCDM7-α(1,3/1,4)FT is able to construct two distinct glycosidic linkages on the subterminal Glc or GlcNAc of type I and type II acceptors, and that this does not depend upon the α(1,2)fucosylation or α(2,3)sialylation status of the terminal galactose on these acceptors. These properties mirror those reported for the fucosyltransferase activities determined by human Lewis blood group locus, and confirm that a single fucosyltransferase can catalyze the formation of two distinct glycosidic linkages.

### The Fucosyltransferase cDNA Identifies Human Genomic Sequences Syntenic to the Human Lewis Blood Group Locus

Genetic date indicate that the human Lewis blood group is determined by a locus on chromosome 19. The fucosyltransferase cDNA was therefore used for Southern blot analysis of a pair of human-mouse somatic cell hybrids that differ only by the presence or absence of human chromosome 19. The results indicate that at high stringency, the fucosyltransferase cDNA identifies cross-hybridizing sequences located on chromosome 19. Taken together with the enzymatic analyses, these data strongly suggest that this cloned cDNA represents the product of the human Lewis blood group locus.

### Experimental Procedures

### cDNA Library Construction

A cDNA library was prepared from poly(A)-plus RNA isolated from human A431 cells, using standard procedures and the mammalian expression vector pCDM7. pCDM7 lacks polyoma sequences present in the vector pCDMS, but is otherwise virtually identical. The library contained 2.6 x 10⁶ independent recombinants.

### Cell Lines

Mouse 3T3-human hybrid cell lines KLEJ-47 and KLEJ-47/P-1 were obtained from Dr. Howard Green (Harvard University, Boston). Mouse 3T3 cells were from Dr. Vishva Dixit (University of Michigan, Ann Arbor). The origins of all other cell lines, and conditions for cell growth are as previously described in the literature.

### Preparation of Panning Dishes

Panning dishes were prepared by first coating them with goat anti-mouse IgM, and then with monoclonal anti-SSEA-1 antibody (ascites generously provided by D. Solter, diluted 1:1000).

### cDNA Library Screening

The A431 library was screened as described previously. Plasmid DNA was rescued from transfected COS-1 cells adherent to panning dishes and introduced into the bacterial host MC1061/P3 by transformation. Transformants were grown to saturation in liquid culture under antibiotic selection, aliquots were removed for frozen storage, and the remainder of the culture was used to prepare plasmid DNA. A portion of this plasmid DNA was used for subsequent enrichment by transfection and immunoselection on anti-SSEA-1 panning dishes.

### FACS Analysis

Transfected COS-1 cells were stained with the mouse IgM anti-SSEA-1 monoclonal antibody (1:1000 dilution of ascites) or mouse monoclonal Igm anti-H monoclonal antibody (Chembiomed, Ltd., Edmonton; 10 mg/ml). Cells were then stained with fluorescein-conjugated goat anti-mouse IgM (Sigma; 40 mg/ml) and subjected to analysis by fluorescence activated cell sorting as described previously.

### Northern and Southern Blotting

A431 cell RNA was subjected to Northern blot analysis as previously described. The probe consisted of a 1.7 kb Xhol - Xbal fragment isolated from the 5' end of the cDNA insert in plasmid pCDM7-α(1,3/1,4)FT. This fragment does not contain the portion of this cDNA that exhibits sequence similarity to human Alu sequences. This probe was labelled by nick translation with α[³²P]dCTP to a specific activity of 6 x 10⁸ cpm/µg.

Genomic DNA was prepared and subjected to Southern blotting as described previously. Blots were subjected to a final wash for 30 minutes at 65°C in O.1X SSC, 0.5% SDS. The probe used was identical to the one used for Northern blot analysis except that it was labelled with the random priming method.

### Sequencing

The cDNA insert in plasmid pCDM7-α(1,3/1,4)FT was sequenced by the chain termination method using a double stranded plasmid DNA template and commercial reagents (Pharmacia). Both strands were sequenced using 17-mer or 19-mer oligonucleotide probes synthesized according to the sequence of the cDNA insert. The DNA sequence was assembled and analyzed using the Beckman Microgenie package, and the Sequence Analysis Software Package of the University of Wisconsin Genetics Computer Group.

### In Vitro Transcription-Translation

Plasmid pCDM7-α(1,3/1,4),FT DNA was linearized downstream from the cloned cDNA insert by digestion with Notl. Capped RNA transcripts were then generated from this linearized template using a T7 polymerase promoter based in vitro transcription kit (Pharmacia). Transcripts initiate from the T7 promoter proximal to the cDNA cloning site in pCDM7. RNA transcripts produced in vitro were used to program a rabit reticulocyte lysate in vitro translation system (Promega), in the presence of ³⁵S-methionine (Amersham), according to the manufacturer's instructions. Membrane-associated radiolabelled in vitro translation products, generated in the presence of canine pancreatic microsomal membranes (Promega) (co-translation) or generated prior to the addition of microsomes (post-translational microsome addition), were isolated from the bulk of the soluble reaction components by centrifugation through a sucrose cushion (0.5 M sucrose, 10 mM Tris 7.4, 150 mM NaCl; 170,000 x g for 60 mm). For endoglycosidase H digestions, pellets containig microsome-associated radiolabelled products were first resuspended in 50 mm sodium citrate pH 5.5, were made 0.2% in SDS, and were heated to 100° C for 4 minutes. Aliquots of this material were then diluted with an equal volume of water and subjected to digestion with either 10 mU or 5 mU of endoglycosidase H for 20 hrs at 37° C, in the presence of 0.1% BSA, 0.5% Triton X-100, 0.5 mM PMSF, 40 µg/ml Bestatin, 10 µg/ml α₂ macroglobulin, and 30 µg/ml of E-64. Alternatively, the pellets were resuspended in ice cold in vitro translation buffer containing 5 mM CaCl2, and were subjected to incubation with 150 µg/ml proteinase K, on ice for 1 hour, in the presence or absence of 1% Triton X-100. The various radiolabelled in vitro translation products were then denatured and reduced by heating to 100° C for 4 minutes in 62.5 mM Tris pH 6.8, 100 mM dithiothreitol, 2% SDS, 10% glycerol, and 0.02% bromphenol blue. Sample were then fractionated through SDS polyacrylamide gels, and the gels were subjected to autoradiography.

### Fucosyltransferase Assays

Cultured cells were washed in PBS, harvested by scraping with a rubber policeman, washed again in PBS, and pelleted by centrifugation. Cell extracts were prepared by resuspending cell pellets in 1% Triton X-100 such that the final protein concentration in the extracts was approximately 5 mg/ml (BCA method, Pierce Chemical Co.).

Fucosyltransferase assays were performed in 50 mM MOPS pH 6.5, 25 mM MnCl₂, 10 mM L-fucose, 5 mM ATP, 3 mM GDP-[¹⁴C]fucose (specific activity of 600,000 cpm/nmol; 35,000 cpm per assay), 2.5 mM acceptor (e.g. 2'-fucosyllactose, N-acetyllactosamine, lactose or lacto-N-biose I), and up to 10 µl of cell extract, final volume of 20 µl. When determining α(1,3)FT specific activities achieved during the sib selection process, and in the analysis of the proteinA-fucosyltransferase fusion protein experiments, the amount of added cell extract and incubation times were adjusted to yield (linear) reaction rates reflecting accurate specific activities. Reactions were incubated at 37° C for 2 or 6 hours, and then terminated by the addition of 20 µl of ethanol, followed by dilution with 500 µl of H₂O. The terminated, diluted reactions were then centrifuged at 15,000 x g for 5 min. Fifty µl of each reaction supernatant was counted to determine total radioactivity, and 200 µl of each was fractionated by Dowex-1 chromatography. The neutral radiolabelled material eluting from the column was then counted directly as a measure of product formation. Enzyme specific activity is defined as pmol of fucose transferred from GDP-fucose to acceptor per mg cell extract protein per hour. Neutral products were also further analyzed as described below by descending paper chromatography and by HPLC to confirm their identity. Parallel reactions were done in the absence of added acceptor to allow correction for transfer to endogenous acceptor molecules and for substrate and product hydrolysis. These control experiments indicated that less than 2.6% of the radioactivity in GDP-[¹⁴C]fucose was found as a neutral product in the absence of added acceptor, and that virtually all of this material represented [¹⁴C] fucose.

In instances where radiolabelled, H type I and H type II molecules were used as acceptors, nonradiolabelled GDP-fucose was included instead of GDP-[¹⁴C]fucose, and reactions were allowed to proceed for 16 hours. Residual unreacted neutral radiolabelled acceptor substrate, and neutral radiolabelled product were isolated by Dowex-1 chromatography and then analyzed by HPLC.

### Preparation of Radiolabelled H Type I and H Type II Acceptors

Cell extracts containing a human α(1,2)FT activity were used to synthesize radiolabelled type I H or type II H acceptor molecules. The cell extracts were prepared from mH1-12 cells, a mouse L cell transfectant containing a human DNA segment that encodes a human α (1,2) FT. These extracts contain no detectable α(1,3)FT activity or α(1,4)FT activity. Lacto-N-biose I (20 mM), or N-acetyllactosamine (20 mM), were incubated with 100 µg of mH1-12 extract protein in 40 µl of 25 mM sodium cacodylate pH 6.2 containing 3 µM GDP-[¹⁴C]fucose, for 16 hours at 37° C. Reactions were terminated by the addition of 40 µl of ethanol followed by dilution with 200 µl of water. Precipitated protein was removed by centrifugation at 12,000 x g for 5 minutes, and the neutral radiolabelled reaction products in the supernatant were isolated by Dowex-1 chromatography. The type I H trisaccharide molecules (lacto-N-biose I reaction) or type II H trisaccharide molecules (N-acetyllactosamine reaction) comprising the majority of the respective neutral radiolabelled materials were then purified by HPLC as described below.

### Product Analysis by HPLC and Descending Paper Chromatography

Neutral radiolabelled reaction products generated by affinity-purified protein A-fucosyltransferase fusion protein, or by pCDM7-α(1,3/1,4FT)-programmed COS-1 extracts, type I or type II disaccharide acceptors, and GDP-[¹⁴C]fucose (see above, Fucosyltransferase Assays) were fractionated by descending paper chromatography or by HPLC chromatography to determine their structures. Samples anaylzed by HPLC were dissolved in 70% acetonitrile and applied to a Dynamax 60A (primary amine column, Rainin Instruments, 4.14 mm x 25 cm) equilibrated in acetonitrile:water (70:30). Compounds were eluted with a linear gradient of acetonitrile:water (70:30 to 40:60), in 1 hour, at a flow rate of 1 ml per minute. The eluant was monitored with a Beckman Instruments on-line radioisotope detector.

Samples analyzed by descending paper chromatography were dissolved in water and fractionated through Whatman No. 40 in phenol/isopropanol/formic acid/water (85:5:10:100, lower layer). After chromatography (Figure 6; 40 hours in panel A or 48 hours in panel B), air-dried chromatograms were cut into 1 cm strips and the radiolabelled compounds were eluted into 2 ml of water. The radioactivity in each eluate was determined by scintillation counting after mixing with 10 ml of scintillation cocktail. HPLC-purified ¹⁴C-labelled type I and type II H-active trisaccharide standards were prepared as described above for the preparation of ¹⁴C-labelled type I and type II H-active acceptor trisaccharides.

### α-L-Fucosidase Digestion

Neutral, HPLC-purified, radiolabelled fucosyltransferase products were subjected to α-L-fucosidase digestion to confirm the alpha anomeric configuration of the attached fucose. (1,3)[¹⁴C]fucosyl-N-acetyllactosamine, (1,3)[¹⁴C]fucosyl-2'-fucosyllactose, (1,3)[¹⁴C]fucosyllactose, and (1,4)[¹⁴C]fucosyllacto-N-biose I were purified by HPLC, and aliquots of each (10,000 to 20,000 cpms) were digested with 100 mU of α-L-fucosidase (E.C. 3.2.1.51, Boehringer-Mannheim) in 70 µl of 100 mM Na citrate pH 5.5, at 37° C for 22 hrs. The reactions were desalted by Dowex cloumn chromatography and subjected to HPLC analysis using conditions described above. The products of the digestion were identified by comparison to parallel separations of L-[¹⁴C]fucose and [¹⁴C]fucose-labelled acceptors. In each case, quantitative release of L-[¹⁴C]fucose was achieved by α-L-fucosidase digestion.

### pPROTA-α(1,3/1,4)FTC Construction and Analysis

A 1344-bp Smal-BamHl segment of the cDNA insert containing the putative fucosyltransferase catalytic domain was isolated from pCDM7-α(1,3/1,4)FT. This fragment was blunt-ended with the Klenow fragment of DNA polymerase I, and the ends were ligated to kinased double stranded linkers (5' CGGAATTCCG 3'). The ligated fragment was gel purified, digested with EcoRI, and gel purified again. This fragment was inserted at the unique EcoRI site of pPROTA. One plasmid (pPROTA-(1,3/1,4)FT_{c}) containing a single insert in the appropriate orientation was analyzed by DNA sequencing to confirm the predicted sequence across the junctions between the vector, linker, and insert.

Plasmids pPROTA-α(1,3/1,4) FT_{c}, pCDM7-α(1,3/1,4)FT, or pPROTA, (50 µg each) were separately introduced into COS-1 cells (500,000 per 100 mm dish) by DEAE-dextran-mediated transfection. After a 72-hour expression period, the media (10 ml) was harvested from each plate and subjected low speed (300 X G for 8 mm) and high speed (100,000 X G for 1 h) centrifugations. The supernatants were then adjusted to 0.05% Tween 20 and were either assayed directly, or were used in IgG-Sepharose binding studies. IgG-Sepharose or Sepharose 6B were preequilibrated as described by the manufacturer (Pharmacia), and then equilibrated in 10% fetal calf serum in Dulbecco's modified Eagle's medium. (FCS/DMEM). Aliquots of processed supernatants containing known amounts of enzyme activity prepared from transfected COS-1 cells were incubated batchwise with 100 µl of equilibrated IgG-Sepharose or Sepharose 6B, overnight at 4° C. Supernatants were saved for assay ("Flow thru" activity, Table III). The matrices were then washed by centrifugation, 9 times with 1 ml of 50 mM This pH 7.5, 1 mg/ml bovine serum albumin, twice with 1 ml of 20 mM Tris pH 7.5, 5 mM CaCl₂, 0.05% Tween-20, and once with FCS/DMEM. The matrices were then resuspended in an equal volume of FCS/DMEM. This suspension was used directly for assay of α(1,3)FT activity.

### Cloning and expression of a DNA sequence encoding GDP-Fuc: [β-D-Gal(1,4)]-D-GlcNAc α(1,3)-Fucosyltransferase:

Cell culture. The source and growth conditions of COS-1 cells, CHO transfectants, and A431 cells are as previously described (Ernst et al, J. Biol. Chem. (1989) 265:3436-3447;
Rajan et al, J. Biol. Chem. (1989) 264:11158-11167). The human HL-60 cell line was obtained from Dr. Steve Kunkel (University of Michigan, Ann Arbor). HL-60 cells were grown in 10% fetal calf serum and Dulbecco's Modified Eagle's Medium.

Antibodies. The anti-Lex antibody anti-SSEA-1 (Solter et al, Proc. Nat. Acad. Sci. (USA) (1978) 75:5565-5569) (mouse monoclonal IgM as ascites) was used. Anti-H and anti-Lewis a antibodies (mouse monoclonal IgM, antigen affinity purified) were purchased from Chembiomed Ltd. (Edmonton, Alberta). Anti-sialyl Lewis x antibody CSLEX1 (Fukushima et al, Cancer Res. (1984) 44:5279-5285) (mouse monoclonal IgM, HPLC purified) and anti-sialyl Lewis a antibody CSLEA1 (Chia et al, Cancer Res. (1985) 45:435-437) (mouse monoclonal IgG3, ammonium sulfate precipitate) were used. Anti-VIM-2 was obtained from Dr. Bruce Macher (San Francisco State University). A pooled mouse IgG antibody preparation (MsIg) was purchased from Coulter. Fluorescein-conjugated goat antimouse IgM or IgG antibodies were purchased from Sigma.

Human genomic library construction. High molecular weight human genomic DNA was prepared from peripheral blood leukocytes as described previously (Ernst et al (1989)). Genomic DNA was subjected to partial digestion with the restriction endonuclease Sau3A. The partially digested genomic DNA was size fractionated by ultracentrifugation through a sodium chloride gradient. Fractions enriched for DNA fragments between 8 Kb and 20 Kb were ligated to XhoI digested lambda FIX (Stratagene) phage arms that had been partially filled in with dTTP and dCTP to make the ends compatible with the Sau3A fragments. The ligation mixture was packaged in vitro with commercial packaging extracts (Stratagene), titered on E. coli host TAP90 (Patterson et al, Nucl. Acids Res. (1987) 15:6298). Approximately 1.0 X 10⁶ recombinant lambda phage were screened by plaque hybridization. Plaque lifts were prepared using nitrocellulose filters (Schleicher and Schuell) and were prehybridized at 42°C for 16 hours in 50% formamide, 5X SSC, 10X Denhart's solution, and 0.1% SDS. Filters were hybridized for 72 hours at 35°C in prehybridization solution containing 10% dextran sulfate, and 100 micrograms per ml denatured salmon sperm DNA. The probe consisted of a 1.7 Kb XhoI-XbaI fragment isolated from the 5' end of a cDNA insert encoding the Lewis blood group α(1,3/1,4) fucosyltransferase which was labeled with [α-³²P] dCTP. The filters were rinsed three times for 20 minutes each as room temperature in 2X SSC and then once for 40 minutes at 50°C and 1X SSC, 0.5% SDS. Filters were then subjected to autoradiography. Eighteen independent hybridization-positive plaques were identified after 2 additional cycles of plaque hybridization. Phage DNAs were prepared from liquid lysates and were subsequently characterized by restriction endonuclease digestions and Southern blot analyses.

DNA sequence analysis. Phage DNA was digested with PstI and a 3.8 Kb fragment homologous to the human α (1,3/1,4) fucosyltransferase cDNA was gel purified and ligated into the PstI site of pTZ18R. A representative subclone containing a single insert was designated pFT-3. A 970 bp hybridization-positive AvaII-PstI fragment was isolated from insert in pFT-3 and subcloned into pTZ18R. The DNA sequence of the insert in this plasmid was determined by the dideoxy chain determination method using T7 DNA polymerase (Pharmacia LKB Biotechnology, Inc.) and oligonucleotides synthesized according to flanking plasmid sequences and subsequently according to the insert sequence. This sequence data was used to generate additional synthetic deoxynucleotides which were then used to sequence portions of the insert in pFT-3. Sequence analysis was performed using the sequence analysis software package of the University of Wisconsin Genetics Computer Group.

Southern blot analysis. High molecular weight human genomic DNA was prepared from whole peripheral blood. Genomic DNA (10 µg) was digested with restriction endonucleases, fractionated through a 0.8% agarose gel, and subjected to Southern blot analysis. To aid in the comparison of hybridization patterns obtained with different probes, duplicate blots were prepared from identical sets of restriction digests electrophoresed on a single gel. Southern blots were hybridized with the temperature being maintained at 35°C. Southern blots were probed with the 1.7 Kb XhoI-XbaI fragment of plasmid pCDM7-α(1,3/1,4)FT which represents the 5' end of the human cDNA encoding the Lewis α(1,3/1,4)fucosyltransferase enzyme. Alternatively, Southern blots were probed with a 400 bp AvaII-PvuII fragment isolated from the insert in pFT-3. Following hybridization, blots were rinsed twice in 2X SSC 0.5% SDS at room temperature for 10 minutes, washed, and then subjected to autoradiography.

Northern blot analysis. Total RNA was prepared from cultured cells Poly A+ RNA was then isolated from total RNA by oligo dT cellulose column chromatography using commercially supplied columns (Clontech) and procedures supplied by the manufacturer. RNA samples were electrophoresed through 1.0% agarose gels containing formaldehyde and were then transferred to a nylon membrane (Hybond-N, Amersham). Northern blots were prehybridized for 1 hour at 61°C in 1X PE (16), 5X SSC, 1% sodium dodecyl sulfate, and 100 µg/ml sheared salmon sperm DNA. Blots were then hybridized for at least 16 hours at 61°C in the same hybridization solution. The probe was a radiolabelled 400 bp AvaII-PvuII fragment isolated from the insert in pFT-3. Following hybridization, blots were subjected to three, ten minute room temperature rinses in 2X SSC, and then washed for 30 minutes at 62°C in 2X SSC, 0.2% SDS.

Transfection and expression of the insert in pFT-3. The 3.8 Kb PstI insert in plasmid pFT-3 was excised and cloned into the PstI site in the mammalian expression plasmid PCDNA1 (Invitrogen). One plasmid with a single insert in the sense orientation with respect to the plasmid's CMV promoter enhancer sequences was designated pCDNA1-α(1,3)FT, and was used for subsequent analysis.

Construction and radiolabeling of stably transfected CHO cell lines. CHO Ade-C cells were transfected with ScaI-linearized pCDM7-α(1,3)FT, co-precipitated in a 10-fold molar excess over EcoRI-linearized pSV2-Neo. A single, clonal, SSEA-1-positive cell line (CHO-LeReFT) was derived from this population. Cell extracts prepared from CHO-LeReFT contained substantial amounts of α(1,3)fucosyltransferase activity when assayed with the acceptor N-acetyllactosamine.

FACS analysis. COS-1 cells-transfected with plasmid DNAs were harvested 48-72 hours after transfection, and stained with monoclonal antibodies diluted in staining media. Anti-Lewis a and anti-H antibodies (mouse IgM monoclonal; antigen-affinity purified; Chembiomed, Edmonton) were used at 10 µg/ml. Anti-SSEA-1 (mouse monoclonal IgM; ascites) was used at a dilution of 1:1000. Anti-sialyl Lewis x (mouse monoclonal IgM; HPLC purified from ascites) was used at 10 ug/ml. Anti-sialyl Lewis a (mouse monoclonal IgG3; ammonium sulfate precipitate of ascites) was used at a dilution of 1:1000. Control mouse IgG3 antibody (MsIg, Coulter) was used at a concentration of 10 ug/ml. Anti-VIM-2 antibody (mouse monoclonal IgM; ascites) was used at a dilution of 1:200. Cells were then stained with fluorescein isothiocyanate-conjugated goat anti-mouse IgM or IgG, as appropriate, and were then subjected to analysis on a FACScan (Becton-Dickinson).

Fucosyltransferase assays. Cell extracts containing 1% Triton X-100 were prepared from transfected COS-1 cells. Fucosyltransferase assays were performed in a total volume of 20 ul, and contained 50 mM sodium cacodylate, pH 6.2, 5 mM ATP, 10 mM fucose, 20 mM MnCl2, 3 uM GDP-¹⁴ Cfucose, and 5 ul (30 ug protein) of cell extract. Acceptor substrates were added to a final concentration of 20 mM. Reactions were incubated at 37°C for 1 hour and terminated by addition of 20 µl ethanol, followed by addition of 600 ul of distilled water. An aliquot of each reaction (50 µl) was subjected to scintillation counting to determine total radioactivity in the reaction. Another aliquot (200 µl) was applied to a column containing 400 ul of Dowex IX2-400, formate form. The flow through fraction, and 2 ul of a subsequent water elution, were collected and pooled, and an aliquot was subjected to scintillation counting to quantitate incorporation of radioactive fucose into neutral product. Descending paper chromatography was used to confirm the structure of the product formed with the acceptor N-acetyllactosamine. The neutral product in the Dowex column eluate was concentrated by lyophilization, resuspended in a small volume of water, and fractionated through Whatman No. 40 in phenol/isopropanol/formic acid/water (85:5:10:100, lower layer). After chromatography (40 hours), the air-dried chromatogram was cut into 1 cm strips and the strips eluted into 2 ml of water. Radioactivity in each eluate was determined by scintillation counting after mixing with 10 ml of scintillation cocktail.

An affinity-purified, protein A-Lewis fucosyltransferase fusion protein was used to prepare an authentic, radiolabeled Galβ8(1→4)[¹⁴CFucα(1→3)]GlcNAc standard for this analysis. This fusion protein was incubated with 20 mM N-acetyllactosamine, 150 um GDP-[¹⁴C]fucose (sp. act. = 3,800 cpms/nmol), in a standard fucosyltransferase reaction mixture. The neutral, radiolabeled product was purified by amine adsorption HPLC on a Waters Carbohydrate Analysis column, using an isocratic gradient consisting of 70% acetonitrile in water, at a flow rate of 1 ml/min. The product (17 nmol) was subjected to analysis by ¹⁴C NMR (Center for Complex Carbohydrate Research, Athens, GA). The sample warn exchanged repeatedly in D₂O and subjected to NMR analysis at 500 MHz. The proton NMR spectrum was recorded on a Bruker AM500 instrument at 28°C. Chemical shifts are relative to acetate (δ, 1.908 ppm). The structure of the expected trisaccharide Galβ (1→4) [Fucα (1→3) ]GlcNAc, was verified by 500 MHz spectroscopy. The spectrum, recorded in D₂O at 28°C, showed H-1 signals for GlcNAC (α-anomer) at δ≈5.102, for Gal at δ≈4.467 and 4.454 ppm (for the α- and β-anomers, respectively, of the trisaccharide) and for Fuc at δ≈5.102 ppm. The anomeric signal for the β-anomer of GlcNAc was obscured by the residual HOD peak (δ≈4.72 ppm). The methyl signals of the GlcNAc N-acetyl group and the C6 protons of Fuc were observed at δ≈2.032 and 1.178 ppm, respectively. These chemical shifts match those published for the authentic trisaccharide Galβ(1→4)[Fucα(1→3)]GlcNAc.

α-L-Fucosidase Digestion. The neutral, chromatographically-purified radiolabeled fucosyltransferase product was subjected to α-L-fucosidase digestion to confirm the alpha anomeric configuration of the attached fucose. 3-[¹⁴C]fucosyl-N-acetyllactosamine was purified by descending paper chromatography as described above, and an aliquot (7000 cpms) was digested with 40 mU of α-L-fucosidase (E.C. 3.2.1.51, Boehringer-Mannheim) in 20 µl of 100 mM Na citrate pH 5.5, at 37°C for 22 hrs. The reaction was desalted by Dowex column chromatography and subjected to HPLC analysis using conditions described above for preparation of the radiolabeled standard. The product of the digestion was identified by comparison to parallel separations of L-[¹⁴C] fucose and the 3-[¹⁴C]fucosyl-N-acetyllactosamine starting material. Quantitative release of L-[¹⁴C]fucose was achieved by α-L-fucosidase digestion.

Obviously, numerous modifications and variations of the present invention are possible in light of the above teachings. It is therefore to be understood that within the scope of the appended claims, the invention may be practiced otherwise than as specifically described herein.

## Claims

1. A glycosyltransferase DNA sequence comprising :
(i) a DNA sequence encoding at least from amino acid position 63 to amino acid position 361 of sequence (I) of Figure 1 and up to the whole sequence (I), or
(ii) a DNA sequence encoding at least from nucleotide position 2089 to nucleotide position 3156 of sequence (IV) of Figure 4 and up to the whole sequence (IV).

2. The DNA sequence of Claim 1, comprising a DNA sequence encoding at least from amino acid position 63 to amino acid position 361 of sequence (I) and up to the whole sequence (I).

3. The DNA sequence of Claim 1, comprising a DNA sequence encoding at least from nucleotide position 2089 to nucleotide position 3156 of sequence (IV) and up to the whole sequence (IV).

4. A vector comprising one of the DNA sequences of Claim 1.

5. A microorganism transformed with the vector of Claim 4.

6. An antibody to the expression product of the DNA sequence of Claims 1 to 3.

## Patentansprüche

1. Glycosyltransferase-DNA-Sequenz, umfassend:
(i) eine DNA-Sequenz, die die Sequenz (1) der Figur 1 mindestens von Aminosäureposition 63 bis Aminosäureposition 361 und bis zur gesamten Sequenz (I) codiert oder
(ii) eine DNA-Sequenz, die die Sequenz (IV) der Figur 4 mindestens von Nukleotidposition 2089 bis Nukleotidposition 3156 und bis zur gesamten Sequenz (IV) codiert.

2. DNA-Sequenz nach Anspruch 1, umfassend eine DNA-Sequenz, die die Sequenz (I) der Figur 1 mindestens von Äminosäureposition 63 bis Aminosäureposition 361 und bis zur gesamten Sequenz (I) codiert.

3. DNA-Sequenz nach Anspruch 1, umfassend eine DNA-Sequenz, die die Sequenz (IV) der Figur 4 mindestens von Nukleotidposition 2089 bis Nukleotidposition 3156 und bis zur gesamten Sequenz (IV) codiert.

4. Vektor, umfassend eine der DNA-Sequenzen nach Anspruch 1.

5. Mikroorganismus, der mit dem Vektor nach Anspruch 4 transformiert ist.

6. Antikörper gegen das Expressionsprodukt der DNA-Sequenz nach Anspruch 1 bis 3.

## Revendications

1. Séquence d'ADN de glycosyltransférase comprenant :
(i) une séquence d'ADN codant au moins de la position de l'acide aminé 63 à la position de l'acide aminé 361 de la séquence (I) de la figure 1 et jusqu'à la séquence (I) entière, ou
(ii) une séquence d'ADN codant au moins de la position de l'acide aminé 2089 à la position de l'acide aminé 3156 de la séquence (IV) de la figure 4 et jusqu'à la séquence (IV) entière.

2. Séquence d'ADN de la revendication 1, comprenant une séquence d'ADN codant au moins de la position de l'acide aminé 63 à la position de l'acide aminé 361 de la séquence jusqu'à la séquence (I) entière.

3. Séquence d'ADN de la revendication 1, comprenant une séquence d'ADN codant au moins de la position de l'acide aminé 2089 à la position de l'acide aminé 3156 de la séquence (IV) de la figure 4 et jusqu'à la séquence (IV) entière.

4. Vecteur comprenant une des séquences d'ADN de la revendication 1.

5. Micro-organisme transformé par le vecteur de la revendication 4.

6. Anticorps contre le produit d'expression de la séquence d'ADN des revendications 1 à 3.
